(19) Europäisches Patentamt
European Patent Office
Office européen des brevets

(11) Veröffentlichungsnummer : **0 378 067 B1**

(12) # EUROPÄISCHE PATENTSCHRIFT

(45) Veröffentlichungstag der Patentschrift : **31.05.95 Patentblatt 95/22**

(51) Int. Cl.[6] : **G03F 7/004**

(21) Anmeldenummer : **90100003.4**

(22) Anmeldetag : **02.01.90**

(54) **Positiv arbeitendes strahlungsempfindliches Gemisch, enthaltend einen mehrfunktionellen alpha-Diazo-beta-ketoester, Verfahren zu dessen Herstellung und strahlungsempfindliches Aufzeichnungsmaterial enthaltend dieses Gemisch.**

(30) Priorität : **12.01.89 DE 3900736**

(43) Veröffentlichungstag der Anmeldung : **18.07.90 Patentblatt 90/29**

(45) Bekanntmachung des Hinweises auf die Patenterteilung : **31.05.95 Patentblatt 95/22**

(84) Benannte Vertragsstaaten : **DE FR GB**

(56) Entgegenhaltungen : **DE-A- 3 841 571**

(73) Patentinhaber : **HOECHST AKTIENGESELLSCHAFT**
**D-65926 Frankfurt (DE)**

(72) Erfinder : **Wilharm, Peter, Dr.-Dipl.-Chem.**
**Am Scheuerling 3**
**D-6200 Wiesbaden (DE)**
Erfinder : **Merrem, Hans-Joachim, Dr.-Dipl.-Chem.**
**Donnersbergstrasse 6**
**D-6104 Seeheim-Jugenheim (DE)**
Erfinder : **Pawlowski, Georg, Dr.-Dipl.-Chem.**
**Fritz-Kalle-Strasse 34**
**D-6200 Wiesbaden (DE)**
Erfinder : **Dammel, Ralph, Dr.-Dipl.-Chem.**
**Eibenweg 39**
**D-6500 Mainz-Bretzenheim (DE)**

EP 0 378 067 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein positiv arbeitendes strahlungsempfindliches Gemisch enthaltend eine photoaktive Komponente und ein in Wasser unlösliches, in wäßrigalkalischer Lösung lösliches, zumindest aber quellbares Bindemittel.

Photoaktive Diazoderivate enthaltende strahlungsempfindliche Gemische, die für eine Bestrahlung mit energiereicher UV-Strahlung geeignet sind, werden seit einiger Zeit in der Literatur beschrieben.

In der US-A-4,339,522 werden positiv arbeitende strahlungsempfindliche Gemische genannt, die als photoaktive Verbindung ein Diazoderivat der Meldrumsäure enthalten. Diese Verbindung soll für eine Belichtung mit energiereicher UV-Strahlung im Bereich zwischen 200 bis 300 nm geeignet sein. Es zeigte sich aber, daß bei Anwendung dieser Mischungen die photoaktive Verbindung unter den in der Praxis häufig eingesetzten höheren Verarbeitungstemperaturen ausgeschwitzt wird; das strahlungsempfindliche Gemisch verliert seine ursprüngliche Aktivität, so daß reproduzierbare Strukturierungen nicht möglich sind.

In den EP-A-0 198 674 und 0 262 864, in der US-A-4,622,283 sowie in der SU-A-1,004,359 werden 2-Diazo-cyclohexan-1,3-dion- bzw. -cyclopentan-1,3-dion-derivate als photoaktive Verbindungen für strahlungsempfindliche Gemische der beschriebenen Art bereitgestellt. Diese Verbindungen weisen eine geringere Flüchtigkeit auf, zeigen aber dafür, je nach vorliegenden Substitutionsmuster, eine schlechte Kompatibilität im strahlungsempfindlichen Gemisch. Dieses zeigt sich insbesondere bei Trocknung der Schichten durch ein Auskristallisieren der photoaktiven Verbindung, durch deren Unlöslichkeit in praxisgerechten Lösungsmitteln oder durch eine durch Phasenseparation hervorgerufene Schichtinhomogenität.

Weitere, im tiefen UV-Bereich empfindliche und positiv arbeitende photoaktive Verbindungen sind aus der EP-A-0 129,694 und der US-A-4,735,885 bekannt. Die in diesen Dokumenten beschriebenen Verbindungen zeigen den Nachteil, daß die aus diesen bei Belichtung gebildeten Carbene keine für die gewünschte Carbonsäurebildung ausreichende Stabilität in der Matrix aufweisen. Dies führt zu einem unzureichenden Löslichkeitsunterschied zwischen den belichteten und unbelichteten Bereichen im Entwickler und damit zu einer unerwünscht hohen Abtragsrate der unbelichteten Bereiche. Eine mögliche Erklärung für dieses Phänomen geben C.G. Willson et al. in SPIE Vol. 771, "Advances in Resist Technology and Processing IV", 2 (1987).

In der EP-A-0 195 986 werden daher α-phosphorylsubstituierte Diazocarbonylverbindungen als photoaktive Verbindungen vorgeschlagen, da diese eine erhöhte Carbenstabilität aufweisen. In der Praxis dürften solche Verbindungen jedoch nur geringeren Einsatz finden, da potentiell als Dotiermittel verwendbare Atome, wie beispielsweise der in diesen Verbindungen enthaltene Phosphor, bei den Verarbeitungsprozessen peinlichst ausgeschlossen werden müssen.

H. Sugiyama, E. Ebata, A. Mizushima und K. Nate stellen in ihrem Aufsatz "Positive Excimer Laser Resist Prepared with Aliphatic Diazoketones" (SPIE Proc., 920, 51 (1988)) neue α-Diazoacetoacetate vor, die als photoaktive Verbindungen in positiv arbeitenden strahlungsempfindlichen Gemischen, insbesondere bei Verwendung von Strahlung im tiefen UV-Bereich, eingesetzt werden. Da es sich um Derivate der Acetoessigsäure handelt, ist die zur Estergruppe β-ständige Ketogruppe direkt einer endständigen Methylgruppe benachbart. Strahlungsempfindliche Gemische, die die genannten Verbindungen als photoaktive Komponenten aufweisen, zeigen gute Ausbleicheigenschaften, ihre Eigenschaften hinsichtlich der Bilddifferenzierung sind aber schlecht.

Die Aufgabe der Erfindung war es daher, ein positiv arbeitendes strahlungsempfindliches Gemisch, enthaltend eine photoaktive Verbindung mit hoher Empfindlichkeit im tiefen UV-Bereich, bereitzustellen, das die zahlreichen beschriebenen Nachteile nicht aufweist, und eine gute Differenzierung zwischen den belichteten und unbe lichteten Schichtbereichen zuläßt. Darüber hinaus sollen die im strahlungsempfindlichen Gemisch enthaltenen photoaktiven Verbindungen mit den unterschiedlichsten, in der Praxis anwendbaren, Polymeren, insbesondere den Bindemitteln, gut verträglich sein, aus dem strahlungsempfindlichen Gemisch nicht ausschwitzen und eine hohe thermische Stabilität sowie eine praxisgerechte Lichtempfindlichkeit aufweisen.

Die Lösung der Aufgabe erfolgt durch Bereitstellen eines positiv arbeitenden strahlungsempfindlichen Gemisches, enthaltend im wesentlichen eine photoaktive Komponente und ein in Wasser unlösliches, aber in wäßriger alkalischer Lösung lösliches, zumindest aber quellbares Bindemittel, dadurch gekennzeichnet, daß als photoaktive Komponente ein mehrfunktioneller -Diazo-β-ketoester der allgemeinen Formel I verwendet wird,

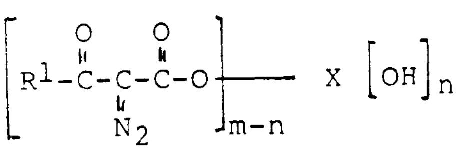

(I)

worin

$R^1$ einen aliphatischen, cycloaliphatischen oder araliphatischen oder aromatischen Rest mit 4 bis 20 Kohlenstoffatomen, wobei einzelne $CH_2$-Gruppen durch Sauerstoff- oder Schwefelatome oder durch

N-

oder NH-Gruppen ersetzt sein können und/oder Ketogruppen enthalten,

X einen aliphatischen, cycloaliphatischen, carbocyclischen, heterocyclischen oder araliphatischen Rest mit 2 bis 22 Kohlenstoffatome, wobei einzelne $CH_2$-Gruppen durch Sauerstoff- oder Schwefelatome oder durch die Gruppen $-NR^2-$, -C(O)-O-, $-C(O)-NR^2-$,

-C(O)-N-,

$-NR^2-C(O)-NR^3-$, $-O-C(O)-NR^2-$,

-O-C(O)-N-,

-O-C(O)-O- oder CH-Gruppen durch

-N-ersetzt

sein können, worin $R^2$ und $R^3$ voneinander unabhängig für Wasserstoff oder einen aliphatischen, carbocyclischen oder araliphatischen Rest stehen,

m eine ganze Zahl von 2 bis 10 und

n eine ganze Zahl von 0 bis 2

bedeuten, mit der Maßgabe, daß

$m-n \geqq 2$ ist.

$R_2$ und $R_3$ bedeuten in der bevorzugten Ausführungsform Wasserstoff, $(C_1-C_3)$Alkyl, $(C_6-C_{12})$Aryl oder $(C_6-C_{11})$Aralkyl, wobei die Reste - insbesondere Aryl oder Aralkyl - am Kern durch Alkyl, Alkoxy, Halogen oder Amino ebenso substituiert sein können. Besonders bevorzugt bedeuten $R_2$ und $R_3$ Wasserstoff oder $(C_1-C_3)$Alkyl, insbesondere aber Wasserstoff.

Die für $R^1$ und X stehenden Reste können ggf. substituiert sein, insbesondere durch $(C_1-C_3)$Alkyl, $(C_1-C_3)$ Alkoxy, Halogen, Amino oder Nitro. Bevorzugt sind Reste $R^1$ und X, die substituiert sind durch $(C_1-C_3)$Alkyl oder $(C_1-C_3)$Alkoxy. Insbesondere wenn $R^1$ und X für einen Alkylrest stehen, werden die unsubstituierten Derivate bevorzugt.

Die aliphatischen Reste $R^1$ können sowohl geradkettig als auch verzweigt sein. Die Zahl der Kettenglieder beträgt hierbei bevorzugt 4 bis 10, insbesondere 4 bis 8. Hierunter fallen die besonders bevorzugten reinen Kohlenstoffketten wie auch die substituierten, in denen $CH_2$-Gruppen durch Sauerstoffatome oder -NH-Gruppen ersetzt und/oder Ketogruppen enthalten sind, worunter Ether-, Keto-, Ester-, Amido- oder Imidogruppen fallen, d.h. auch Ester der Carbaminsäure. In den reinen aliphatischen Resten $R^1$ treten Ethergruppen besonders bevorzugt zweimal pro Rest $R^1$ auf. Sofern es sich um reine, insbesondere geradkettige, Kohlenstoffketten handelt, ist eine Begrenzung der Kohlenstoffzahl nicht zwingend; es können durchaus aliphatische Reste mit bis zu 20 Kohlenstoffatomen zum Einsatz kommen. Besonders bevorzugt ist dennoch der t-Butylrest.

Sofern $R^1$ ein cycloaliphatischer Rest bedeutet, ist die Zahl der Ringglieder bevorzugt 4, 5, 6 oder 12, insbesondere 4, 5 oder 6. Die unsubstituierten Varianten werden besonders bevorzugt. Als Beispiele gelten der Cyclobutyl-, Cyclopentyl- und der Cyclohexylrest. Besonders bevorzugt ist der Cyclohexylrest.

Ist $R^1$ ein araliphatischer Rest, so ist die Zahl der Glieder des aliphatischen Teils 2 bis 11, insbesondere 2 bis 5. Sofern es sich um eine reine Kohlenstoffkette im aliphatischen Teil handelt, ist die Anzahl der C-Atome bevorzugt 1 oder 2. Werden $CH_2$-Gruppen durch Sauerstoffatome ersetzt, so können diese als Brückenglied zwischen aromatischem und aliphatischem Teil des Rests $R^1$ vorkommen, aber auch im aliphatischen Teil. In beiden Fällen ist es besonders bevorzugt, wenn die verbleibende Gesamtzahl der Kohlenstoffatome als Kettenglieder im aliphatischen Teil dieses Restes 1 oder 2 beträgt, wobei das Ether-Sauerstoffatom im Falle von 2 Kohlenstoffatomen als Kettenglieder in der Weise angeordnet ist, daß es von beiden $CH_2$-Gruppen benachbart ist. Hierunter sind zu nennen der Benzyl-, der Phenoxymethylen- und der Benzyloxymethylenrest. Werden zusätzlich noch weitere $CH_2$-Gruppen im aliphatischen Teil des araliphatischen Restes $R^1$ durch Heteroatome ersetzt und/oder Substitutionen an diesem vorgenommen, so ist die Gesamtzahl der Kettenglieder des aliphatischen Teils 3 bis 5. Hierunter fallen u.a. über Estergruppen gebundene Phenyl- oder Benzylreste, aber auch die Benzyl- oder Phenylester der Carbaminsäure. Der aliphatische Teil kann aber auch die Imidogruppe einer

aromatischen Dicarbonsäure darstellen. Der aromatische Teil eines solchen Restes besteht insbesondere aus 6 C-Atomen. Wenn der aromatische Teil des araliphatischen Restes direkt benachbart zur Ketogruppe steht, d.h. als Arylenrest eingebunden ist, so gelten für den darin vorkommenden aliphatischen Teil keine Beschränkungen hinsichtlich der Zahl mindestens vorkommender C-Atome.

Die aromatischen Reste $R^1$ weisen bevorzugt keine Heteroatome wie z.B. Sauerstoff in ihrem Ringsystem auf. Ist $R^1$ ein aromatischer Rest, so enthält er bevorzugt 6 bis 12 C-Atome, insbesondere 6 C-Atome, d.h. entspricht einem Phenylrest. Aromatische Reste $R^1$ sind aber nicht bevorzugt.

Insgesamt sind als besonders bevorzugte Reste $R^1$ t-Butyl, n-Hexyl, Nonyl, Octadecyl, 2,5-Dioxahexyl, Cyclopentyl, Cyclohexyl, Benzyl, Phenoxymethyl und Benzyloxymethyl zu nennen. Insbesondere bevorzugt ist der t-Butyl-, Phenoxymethyl- und der Cyclohexylrest.

X steht für einen aliphatischen oder cycloaliphatischen Rest, der gesättigt wie auch ungesättigt sein kann, einen carbocyclischen, heterocyclischen oder araliphatischen Rest mit 2 bis 22 Kohlenstoffatomen, insbesondere mit 2 bis 17 Kohlenstoffatomen. In diesen Resten kann zudem mindestens eine $CH_2$-Gruppe durch Heteroatome wie Sauerstoff, Schwefel oder durch Gruppen wie $-NR^2-$, -C(O)-O-, $-C(O)-NR^2-$,

$$-C(O)-\underset{|}{N}-,$$

$-NR^2-(O)-NR^3-$, $-O-C(O)-NR^2-$,

$$-O-C(O)-\underset{|}{N}-$$

oder -O-C(O)-O- oder CH-Gruppen durch -N- ersetzt sein. Insbesondere bevorzugt sind bei den aliphatischen oder araliphatischen Resten diejenigen Varianten, in denen maximal zwei $CH_2$-Gruppen durch einen Typ der oben genannten Gruppen ersetzt worden sind. Werden $CH_2$-Gruppen durch Heteroatome ersetzt, so kann die Anzahl dieser bevorzugt maximal 5, insbesondere maximal 3 sein. Dabei ist besonders bevorzugt, wenn alle zu ersetzenden $CH_2$-Gruppen durch Heteroatome eines Typs ersetzt werden.

Handelt es sich um einen unsubstituierten, gesättigten oder ungesättigten aliphatischen Rest X, so enthält dieser in der bevorzugten Variante maximal 6 Kohlenstoffatome. Zu den ungesättigten aliphatischen Resten X zählen insbesondere solche, deren $CH_2$- bzw. CH-Gruppen nicht durch Heteroatome bzw. den obengenannten Gruppen ersetzt sind. Sie enthalten in ihrer besonderen Ausführungsform maximal eine C-C-Mehrfachbindung; die Zahl der Kettenglieder ist in einem solchen Rest besonders bevorzugt 4. Die genannten Reste X können sowohl 2- wie auch 3-bindig sein, bevorzugt ist allerdings, wenn X 2-bindig ist.

Werden in die aliphatischen Reste X $CH_2$-Gruppen durch Heteroatome ersetzt, so werden diese bevorzugt von Alkylenresten mit jeweils mindestens 2 $CH_2$-Gruppen eingebunden. Ist Schwefel das Heteroatom im aliphatischen Rest X, so kommt dieser besonders bevorzugt pro Rest lediglich einmal vor. Er ist insbesondere von Alkylresten mit jeweils maximal 3 $CH_2$-Gruppen umgeben. Wird Sauerstoff als Heteroatom im aliphatischen Rest eingesetzt, so kann dieser häufiger pro Rest vorkommen, insbesondere 2 bis 4 mal. In diesem Fall enthalten die Alkylenreste, in die zwei Sauerstoffatome eingebunden sind, mindestens 3 $CH_2$-Gruppen.

Ist die Anzahl der Kohlenstoffatome im unsubstituierten aliphatischen Rest größer als 3, so liegt dieser Alkylenrest insbesondere als verzweigtes Isomeres vor. Insbesondere bevorzugt ist der t-Butyl- oder der t-Pentylrest.

Im Rest X können auch mehrere Alkylreste, insbesondere der t-Butyl- oder t-Pentylrest, über Heteroatome oder $CH_2$-Gruppen ersetzende Gruppen, die oben genannt sind, verbunden sein. Dies ist dann besonders bevorzugt, wenn diese Reste mehr als zweibindig sind:

Sofern die t-Butyl oder t-Pentylreste dreibindig sind, ist m mindestens 3. m = 4 wird bevorzugt erreicht, indem ein zweibindiger t-Pentyl- oder t-Butylrest im Rest X zweimal vorliegt. m = 4 wird auch erreicht, indem ein zweibindiger Propylrest am Rest X zweimal vorkommt.

m = 6 wird bevorzugt erreicht, indem zwei dreibindige Reste (t-Butyl oder t-Pentyl) oder drei zweibindige Propylreste im Rest X vorliegen. m = 8 wird z.B. erreicht durch die Kombination von 4 zweibindigen Resten oder von 2 dreibindingen Resten mit einem zweibindigen Rest.

Die mehr als Zweibindigkeit des Restes X kann auch dadurch erreicht werden, daß mehr als zweibindige Heteroatome enthalten sind: Wird im aliphatischen Rest X eine CH-Gruppe durch

$$-\underset{|}{N}-$$

ersetzt, so können m-Werte von 2 bis 3 erreicht werden. Werden zwei CH-Gruppen durch

$$-\underset{|}{N}-$$

ersetzt, so kann m maximal 4 erreichen; sofern diese Teil eines cycloaliphatischen Ringes darstellen, ist m maximal 2.

In allen Fällen, in denen im Rest X CH-Gruppen durch

$$-\underset{\backslash}{N}-$$

ersetzt werden, findet bevorzugt keine Substitution einer $CH_2$-Gruppe durch ein weiteres Heteroatom oder durch eine der obenbeschriebenen Gruppen statt. Die Anzahl oer $CH_2$-Gruppen, die zwischen dem Stickstoffheteroatom und dem an den Rest X gebundenen Rest nach der allgemeinen Formel I liegen, beträgt mindestens 2; insbesondere ist diese die Zahl 2.

Reine cycloaliphatische Reste, d.h. unsubstituierte, als Varianten des Restes X sind nicht bevorzugt. Als cycloaliphatischer Rest wird insbesondere der Cyclohexylrest genannt. Dieser kann aber substituiert sein, insbesondere durch Hydroxyl und/oder Alkyl oder Alkylen, wobei dessen Bindigkeit (d.h. m) bevorzugt durch die Anzahl der Alkylensubstituenten am cycloaliphatischen Rest bestimmt wird. Ganz besonders bevorzugt ist ein Cyclohexylrest, der vier Methylengruppen als Substituenten enthält, die gleichzeitig die Bindung der -Diazo-β-ketoester-Einheiten nach der allgemeinen Formel I gewährleistet (m = 4). Besonders bevorzugt ist, wenn dieser Rest X noch eine Hydroxylgruppe, insbesondere eine sekundäre Hydroxylgruppe, enthält, so daß n = 1 ist.

Zumeist handelt es sich bei den cycloaliphatischen Resten als Variante des Restes X mehr um eine Kombination aus einem cycloaliphatischen und einem kettenförmigen aliphatischen Teil. In diesem Fall ist der cycloaliphatische Teil bevorzugt nicht in der Weise substituiert, daß $CH_2$-Gruppen aus diesem Teil durch Heteroatome oder durch Gruppen aus der obengenannten Reihe ersetzt sind.

Als eine Ausnahme gilt ein sechsgliedriger Ring (Hete rocyclus) aus drei Carbonsäureamideinheiten, in dem die Koppelung der α-Diazo-β-ketoester-Einheiten der allgemeinen Fromel I über Ethylengruppen an den Amidostickstoff erfolgt. m ist daher 3 und n ist 0.

Wenn X aber eine Kombination aus einem reinen cycloaliphatischen Teil und einem oder mehreren kettenförmigen aliphatischen Teilen mit 2 oder mehreren Kohlenstoffatomen ist, so ist der cycloaliphatische Teil insbesondere direkt benachbart zu einer $CH_2$-Gruppe, die durch eine der obengenannten Heteroatome oder Gruppen ersetzt ist. Besonders bevorzugt ist diejenige Variante, in der der cycloaliphatische Teil direkt benachbart ist zu einem Stickstoffatom, insbesondere zu den Gruppen

$$-NH-\overset{O}{\overset{\|}{C}}-O- \text{ und } -NH-\overset{O}{\overset{\|}{C}}-NH-.$$

In diesem Fall wird als cycloaliphatischer Teil bevorzugt ein Cyclohexylrest eingesetzt, der sowohl einbindig wie auch zweibindig sein kann, letzteres bevorzugt in 1,4-Position. In beiden Fällen erfolgt die Verknüpfung einer der freien Valenzen der Reste

$$-NH-\overset{O}{\overset{\|}{C}}-O- \text{ oder } -NH-\overset{O}{\overset{\|}{C}}-NH-$$

mit der -Diazo-β-ketoester-Einheit nach der allgemeinen Formel I über ein Alkylenrest mit mindestens 2 $CH_2$-Einheiten. Sofern es sich um einen

$$-NH-\overset{O}{\overset{\|}{C}}-NH-Rest$$

handelt, ist als verknüpfende Gruppe bevorzugt ein t-Butylenrest formuliert. Sofern es sich um einen

$$-NH-\overset{O}{\overset{\|}{C}}-O-Rest$$

handelt, d.h. die diskutierte Verknüpfung über das Sauerstoffatom dieser Gruppe erfolgt, wird bevorzugt ein Ethylenrest als verknüpfende Gruppe eingesetzt.

Handelt es sich um einen araliphatischen Rest als Variante der Gruppe X, so kann der aromatische Teil, insbesondere ein Phenyl-, bzw. wenn Zweibindigkeit vorliegt, ein Phenylenrest, sowohl über ein Stickstoffatom wie auch über ein Sauerstoffatom gebunden sein. Bevorzugt ist allerdings auch hier - wenn beide Atome zur Auswahl stehen - das Stickstoffatom. Als Beispiel, in dem der aromatische Teil direkt über ein Sauerstoffatom gebunden ist, zählt ein über eine Ethylengruppe an die α-Diazo-β-Ketoester-Einheit gebundenes Ethersauerstoffatom. Als dritte Variante ergibt sich in diesem Fall noch die Möglichkeit, daß der aromatische Rest, insbesondere wenn er einbindig ist, über die Ketogruppe eines Restes

$$-\overset{O}{\overset{\|}{C}}-NH-$$

gebunden vorliegt. Das Stickstoffatom trägt in diesem Fall insbesondere einen Ethylenrest.

Im Falle des Vorliegens eines araliphatischen Restes X ist der über das Stickstoffatom eines eine $CH_2$-Gruppe ersetzenden Restes

$$-NH-\overset{O}{\overset{\|}{C}}-NH- \quad oder \quad -O-\overset{O}{\overset{\|}{C}}-NH-$$

gebundene aliphatische Teil des Restes X insbesondere ein t-Butylen- oder ein Ethylenrest; ist der aliphatische Teil über das Sauerstoffatom gebunden, so ist dieser besonders bevorzugt ein Ethylenrest.

Daß über das Sauerstoffatom der obengenannten, eine $CH_2$-Gruppe im Rest X zu ersetzenden Gruppen bevorzugt eine Ethylengruppe gebunden ist, läßt sich auch auf aliphatische Reste im allgemeinen übertragen, während über das Stickstoffatom sowohl Ethylenreste wie auch höhere aliphatische Reste, insbesondere aus Kohlenstoffketten mit mehr als 3 Kohlenstoffatomen, gebunden sind. Bevorzugt ist der t-Butylenrest.

Sofern es sich um araliphatische und aliphatische Reste X handelt, werden bevorzugt maximal 2 $CH_2$-Gruppen durch Reste wie

$$-NH-\overset{O}{\overset{\|}{C}}-NH- \quad oder \quad -O-\overset{O}{\overset{\|}{C}}-NH-$$

ersetzt. Der Rest

$$-\overset{O}{\overset{\|}{C}}-\underset{|}{N}-$$

als Ersatz für ein CH-Gruppe liegt in der besonders bevorzugten Ausführungsform nur einmal in einem Rest X vor.

In den oben dargestellten Varianten der Reste $R^1$ und X ist m bevorzugt eine ganze Zahl von 2 bis 8 und n eine ganze Zahl von 0 bis 2.

Besonders bevorzugt ist m eine ganze Zahl von 2 bis 6 und n = 0.

Die vorstehend näher charakterisierten, im erfindungsgemäßen positiv arbeitenden strahlungsempfindlichen Gemisch eingesetzten α-Diazo-β-ketoester der allgemeinen Formel I, sind als photoaktive Komponenten hervorragend geeignet. Insbesondere zeigen diese Verbindungen eine hohe Absorption bei Belichtung mit Strahlung einer Wellenlänge von 190 bis 350 nm, bevorzugt von 200 bis 300 nm. Die mehrfunktionellen Verbindungen nach der allgemeinen Formel I sowie deren Herstellung werden in der gleichzeitig eingereichten deutschen Patentanmeldung P 39 00735.9 (Hoe 89/K 002) beschrieben.

Beim Verfahren zur Herstellung der -Diazo-β-ketoester hat sich als besonders günstig erwiesen, zunächst geeignete Vorstufen für β-Ketoester der allgemeinen Formel II zu synthetisieren und diese in einer Folgereaktion durch einen sogenannten Diazotransfer in die -Diazo-β-ketoester der Formel I zu überführen (vgl. M. Regitz et al., Org. Prep. Proced., 1, 99 (1969)):

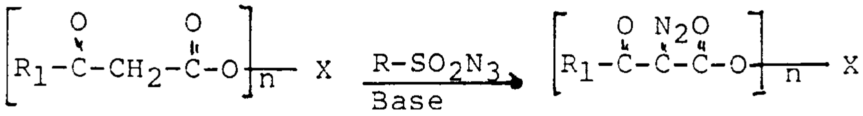


(II) (I) Schema 1

Zu diesem Zweck wird ein β-Ketoester der allgemeinen Formel II (in der $R_1$ und X die in Formel I angegebene Bedeutung aufweisen) in der 5 bis 50fachen, bevorzugt 10fachen Menge (bezogen auf das Gemisch) eines geeigneten Lösungsmittel gelöst und die Lösung auf eine Temperatur zwischen -15° C bis +15° C, bevorzugt -5° C bis +5° C gekühlt. Als Lösungsmittel geeignet sind Alkohole, wie Methanol, Ethanol, Alkoholether wie Ethylenglykolmonomethylether, chlorierte Kohlenwasserstoffe, wie Dichlormethan oder Trichlormethan oder bevorzugt aliphatische Nitrile, wie Acetonitril oder Gemische dieser Lösungsmittel. Besonders werden dabei solche bevorzugt, die einen Siedepunkt zwischen 30° C und 140° C aufweisen. Die eigentliche erfindungsgemäße Umsetzung kann nach 3 Varianten durchgeführt werden.

Variante A:

Die gekühlte Lösung wird mit dem 1- bis 1,3-fachen Überschuß - bezogen auf die Anzahl der umzusetzenden aktivierten Methylengruppen - eines Diazotransferreagenzes versetzt. Als Transferreagenzien haben sich insbesondere aromatische und aliphatische Sulfonylazide, wie Toluolsulfonylazid, 4-Carboxyphenylsulfonylazid, 2-Naphthalinsulfonylazid oder Methylsulfonylazid bewährt. Bezogen auf das Sulfonylazid wird dann die äquimolare Menge einer Base, bevorzugt eines tertiären Amins, zu der Lösung hinzugefügt. Die Temperatur der Mischung muß dabei konstant gehalten werden. Bevorzugte Amine sind beispielsweise Triethylamin, Triisopropylamin oder Diaza-bicyclo[2.2.2]octan. Besonders bevorzugt ist die Verwendung von Triethylamin als Base. Die erhaltene Mischung wird 5 bis 50 Minuten, bevorzugt 10 bis 15 Minuten, bei der vorgegebenen Temperatur gerührt, auf Raumtemperatur erwärmt und dabei weitere 1 bis 24 Stunden, bevorzugt 2 bis 4 Stunden, gerührt. Je nach Art des eingesetzten Sulfonylazids kann das dabei gebildete Sulfonylamid ausfallen, so daß es nach Beendigung der Reaktion ggf. abfiltiert wird.

Variante B:

Alternativ zu Variante A können auch der β-Ketoester der allgemeinen Formel II und das Amin unter den vorstehend beschriebenen Bedingungen vorgelegt werden und das Sulfonylazid anschließend unter Beibehaltung der Temperatur zudosiert werden.

Variante C:

Als besonders günstig hat sich jedoch eine modifizierte Variante A erwiesen, in der die Lösung des β-Ketoesters der allgemeinen Formel II zunächst nur mit dem 0,7 bis 0,9-fachen Überschuß - bezogen auf die Anzahl der umzusetzenden aktivierten Methylengruppen - eines Sulfonylazids, bevorzugt Toluolsulfonylazid, versetzt wird und unter Beibehaltung der vorgegebenen Temperatur die Gesamtmenge des Amins zusetzt wird. Die Mischung wird dann ggf. unter Erwärmung auf Raumtemperatur gerührt. Nach etwa 10 - 120 Minuten ist das Toluolsulfonylazid chromatographisch nicht mehr nachweisbar. Hierauf wird die Mischung ggf. erneut gekühlt und diesmal mit dem 0,6 bis 0,1-fachen Überschuß von 4-Carboxyphenylsulfonylazid versetzt, so daß ein Gesamtüberschuß an Sulfonylazid entsprechend der Variante A entsteht. Die Rohprodukte, die nach dieser Variante hergestellt werden, zeigen eine hohe Reinheit.

Das nach den Varianten A bis C erhaltene Gemisch wird vom Lösungsmittel und überschüssigen Reagenzien befreit und in einem inerten, nicht mit Wasser mischbaren Lösungsmittel, insbesondere Dichlormethan oder Diethylether, aufgenommen. Die Mischung wird zur Entfernung von Sulfonylamidresten zweimal mit 5%iger Kalilauge und anschließend mit Wasser neutral gewaschen, über einem geeigneten Trocknungsmittel getrocknet und erneut vom Lösungsmittel befreit. Der verbleibende Rückstand, insbesondere bei Anwendung der Variante C fast ausschließlich aus reinem α-Diazo-β-ketoester der allgemeinen Formel I bestehend, kann nach bekannten Methoden, beispielsweise mittels Kristallisation oder Chromatographie, aufgearbeitet werden.

Die Herstellung der zur Umsetzung zu den α-Diazo-β-ketoestern der allgemeinen Formel I erforderlichen β-Ketoester der allgemeinen Formel II kann auf unterschiedliche, in der Literatur bekannte, Verfahrensweisen erfolgen:

1. Entsprechend dem Schema 2 erfolgt eine Umsetzung eines monofunktionellen 5-Acyl-2,2-dialkyl-1,3-dioxan-4,6-dions (5-Acyl-meldrumsäure) der allgemeinen Formel III mit einem mehrwertigen Alkohol der allgemeinen Formel IV zum mehrfunktionellen β-Ketoester der allgemeinen Formel II. Die Herstellung von 5-Acyl-meldrumsäure-derivaten der allgemeinen Formel III und deren Umsetzung zu β-Ketoestern der allgemeinen Formel II ist für monofunktionelle Verbindungen bekannt und kann beispielsweise analog zu den Vorschriften von Y. Oikawa et al., J. Org. Chem., 43, 2087 (1987) durch Umsetzung von Säurechloriden mit Meldrumsäure, oder analog zu Vorschrift von P. Houghton und D.J. Lapham, Synthesis, 1982, 451ff, erfolgen. Die Produkte werden in ihrer Enolform isoliert.

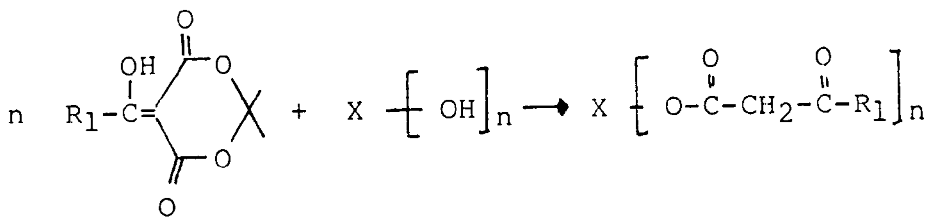

(III) (IV) (II)

Schema 2

2. Entsprechend dem Schema 3 erfolgt die Umsetzung eines monofunktionellen β-Ketoesters, bevorzugt eines Methyl- oder Ethylesters der allgemeinen Formel V, mit einem mehrwertigen Alkohol der allgemeinen Formel IV zum mehrfunktionellen β-Ketoester der allgemeinen Formel II. Die Umesterungsreaktion zur Herstellung von monofunktionellen β-Ketoestern der allgemeinen Formel II ist bekannt und wird von A.R. Bader et al. in J. Amer. Chem. Soc., 73, 4195ff (1951) beschrieben.

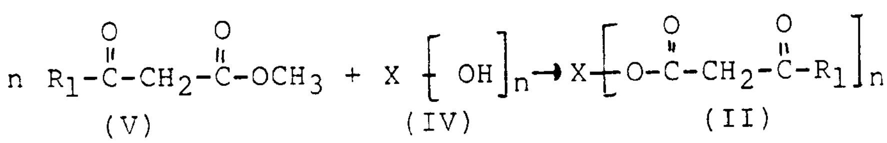

(V) (IV) (II)

Schema 3

Bei der Reaktionsfolge nach Schema 2 wird das betreffende Derivat der 5-Acyl-meldrumsäure der allgemeinen Formel III mit der zum gewünschten Umsetzungsgrad erforderlichen Menge des betreffenden mono- oder polyfunktionellen Alkohols der allgemeinen Formel IV versetzt, und das Gemisch anschließend in der 5- bis 20-fachen, bevorzugt 10-fachen Menge, eines nicht mit Alkoholen oder der Meldrumsäure reagierenden Lösungsmittels, beispielsweise eines Ketons wie Aceton oder Ethylmethylketon, oder eines Ethers, wie 1,2-Dimethoxyethan oder Dioxan, ggf. unter Erwärmung, gelöst. Die klare Lösung wird auf eine Temperatur von 60° C bis 120° C, bevorzugt auf 80° C bis 100° C erwärmt. Die beginnende Umsetzung macht sich durch lebhafte Kohlendioxidentwicklung bemerkbar. Die Mischung wird etwa 1 bis 6 Stunden, bevorzugt 2 bis 3 Stunden, bei der vorgegebenen Temperatur gerührt, bis keine $CO_2$-Entwicklung mehr beobachtbar ist.

Anschließend wird das Lösungsmittel im Vakuum abgetrennt. Obwohl der betreffende β-Ketoester der allgemeinen Formel II in hoher Reinheit anfällt, kann das Produkt ggf. weiterhin nach den durch den Fachmann bekannten Methoden gereinigt werden.

Besonders geeignete Derivate der 5-Acyl-meldrumsäure der allgemeinen Formel III sind solche, in denen $R^1$ Cyclobutyl, Butyl, Pentyl, Cyclopentyl, Hexyl, Cyclohexyl, Heptyl, Octyl, Nonyl oder höhere Alkylreste mit bis etwa 22 Kohlenstoffatomen bedeuten, die ggf. durch weitere Alkylreste, Alkoxyalkylreste, Arylreste, Alkoxyarylreste, Aryloxyarylreste, Halogenatome oder durch andere funktionelle Gruppen, beispielsweise durch endständige Säureesterfunktionen, substituiert sind oder in denen einzelne $CH_2$-Gruppen durch Sauerstoff- oder Schwefelatome, durch Gruppen wie -C(O)-O-, -C(O)-$NR^2$-, -$NR^2$-C(O)-$NR^3$-, -O-C(O)-$NR^2$-, -O-C(O)-O-, -$NR^2$-, worin $R^2$ und $R^3$ die vorstehend beschriebene Bedeutung haben, ersetzt sein können.

Als besonders bevorzugte Reste $R^1$ sind t-Butyl, n-Hexyl, 2,5-Dioxahexyl, Cyclopentyl, Cyclohexyl, Benzyl, Phenoxymethyl und Nonyl genannt. Insbesondere bevorzugt ist der t-Butyl-, Cyclohexyl- und der

Phenoxymethylrest.

Als Alkohole der allgemeinen Formel IV können di-, tri- oder höherfunktionelle Alkohole Verwendung finden; bevorzugt sind Alkohole, die 2 bis 6 OH-Gruppen pro Molekül enthalten. Difunktionelle Alkohole der allgemeinen Formel IV umfassen beispielsweise Ethylenglykol, Propan-1,2-diol, Propan-1,3-diol, 2,2-Dimethyl-propan-1,3-diol, 3-Chlor-propan-1,2-diol, Butan-1,2-diol, Butan-1,3-diol, Butan-2,3-diol, Butan-1,4-diol, 2,3-Dimethyl-butan-2,3-diol, Pentan-1,2-diol, Pentan-1,5-diol, Pentan-2,4-diol, 2-Methyl-pentan-2,4-diol, Hexan-1,6-diol, Hexan-2,5-diol, 2,5-Dimethyl-hexan-2,5-diol, 2-Ethyl-hexan-1,3-diol, Octan-1,8-diol, Decan-1,10-diol, Dodecan-1,2-diol, Dodecan-1,12-diol, Phenylethylenglykol, oder höhere ggf. substituierte und verzweigte Alkandiole, wie Weinsäure, Dimethyltartrat, Diethyltartrat, Diisopropyltartrat, 3-Allyloxy-1,2-propandiol, Dihydroxyaceton, Tetraphenyl-1,2-ethandiol, in denen ggf. eine oder mehrere Methylengruppen durch Sauerstoffatome enthaltende Reste, wie z.B. in Diethylenglykol, Triethylenglykol, Tetraethylenglykol, Dipropylenglykol, durch Schwefelatome enthaltende Reste wie in 2,2′-Thiodiethanol, 1,8-Dihydroxy-3,6-dithiaoctan, oder die Stickstoffatome enthaltende Reste wie beispielsweise in N-Methyl-2,2′-iminodiethanol, N-Butyl-2,2′-iminodiethanol, N-tert.-Butyl-2,2′-iminodiethanol, N-Phenyl-2,2′-diiminodiethanol, oder durch die obenstehend bezeichneten Gruppen substituiert sein können. Darüber hinaus können auch ungesättigte Diole, wie beispielsweise 2-Buten-1,4-diol, 2-Butin-1,4-diol, 3-Hexin-2,5-diol, 2,5-Dimethyl-3-hexin-2,5-diol, oder cyclische Diole, die wiederum durch Sauerstoff-, Schwefel- oder Stickstoffatome substituiert sein können, wie Cyclohexan-1,2-diol, Cyclohexan-1,4-diol, 1,4-Bis(hydroxymethyl)-cyclohexan oder 1,4-Bis(2-hydroxyethyl)-piperazin, mit Vorteil eingesetzt werden. Derartige Diole sind i.a. kommerziell erhältlich. Weitere Diole lassen sich herstellen, beispielsweise durch Umsetzung von Diolen, enthaltend primäre Aminogruppen, beispielsweise von 2-Amino-2-methyl-1,3-propandiol mit monofunktionellen Säurederivaten, wie Carbonsäure- oder Sulfonsäurechloriden, oder monofunktionellen Isocyanaten, aber auch mit difunktionellen Säurederivaten oder entsprechenden Isocyanaten.

Trifunktionelle Alkohole leiten sich bevorzugt vom Glycerin, von höheren α,β,ω-Triolen oder vom Triethanolamin ab, wobei auch hier höherkettige Derivate, insbesondere ethoxylierte Verbindungen und heterocyclische Verbindungen, wie z.B. 1,3,5-Tris(2-hydroxyethyl)-cyanursäure gleichermaßen Verwendung finden können. Genannt werden Glycerin, 2-Hydroxymethyl-2-methyl-propan-1,3-diol, 2-Ethyl-2-hydroxymethyl-propan-1,3-diol, 2,3-Isopropyliden-erythruronsäure, Hexan-1,2,6-triol, 1,1,1-Triethanolamin, 1,1,1-Tripropanolamin, sowie partiell acetalisierte oder ketalisierte Zuckerderivate.

Darüber hinaus lassen sich auch Umsetzungsprodukte aus tetrafunktionellen Alkoholen oder Aminotriolen mit Säurederivaten, Isocyanaten oder cyclischen Carbonaten verwenden. Höherwertige Alkohole der allgemeinen Formel III leiten sich beispielsweise von Kondensationsprodukten des Glycerins, des Pentaerythrits oder von Umsetzungsprodukten difunktioneller Säurederivate oder Isocyanate mit höherwertigen Alkoholen oder Aminoalkoholen ab. Die Liste der verwendbaren Alkohole ist damit durchaus nicht vollständig; es lassen sich praktisch alle Alkohole verwenden, die keine weitere mit Säureestern reagierende Gruppe aufweisen oder die unter den beschriebenen Umsetzungsbedingungen eindeutig unter Esterbildung reagieren.

Bei der Herstellung von β-Ketoestern der allgemeinen Formel II durch Umesterung gemäß Schema 3 werden monofunktionelle β-Ketoester der allgemeinen Formel V mit Alkoholen der allgemeinen Formel IV in der Weise eingesetzt, daß ein 5 bis 200%iger Überschuß, bevorzugt ein 10 bis 50%iger Überschuß eines mit einem niedermolekularen Alkohol veresterten β-Ketoesters, z.B. eines Methyl- oder Ethylesters, bei 100 bis 160° C, bevorzugt bei 120 bis 140° C, umgesetzt wird. Zur Erhöhung der Löslichkeit des Alkohols der allgemeinen Formel IV im β-Ketoester der allgemeinen Formel V kann ggf. ein Lösungsvermittler, wie Dimethylformamid oder N-Methylpyrrolidon, zugesetzt werden. Durch Anlegen eines schwachen Vakuums von 800 bis 20 Torr, bevorzugt von 400 bis 100 Torr, wird das Gleichgewicht durch Abdestillieren des gebildeten niederen Alkohols kontinuierlich in die gewünschte Richtung verschoben. Nachdem die theoretische Menge an niederem Alkohol abdestilliert ist, wird der überschüssige niedrig-veresterte β-Ketoester der allgemeinen Formel V sowie ggf. der zugefügte Lösungsvermittler im Hochvakuum abdestilliert. Als Rückstand verbleibt der häufig ölig anfallende β-Ketoester der allgemeinen Formel II in zumeist sehr hoher Reinheit, so daß er ohne weitere Reinigung für den Diazotransfer verwendet werden kann.

Die bei dieser Reaktionsfolge erforderlichen niedrigveresterten β-Ketoester der allgemeinen Formel V sind z.T. kommerziell verfügbar oder lassen sich nach zahlreichen literaturbekannten Methoden herstellen. Besonders bevorzugt ist dabei ihre Herstellung aus den entsprechenden Derivaten der 5-Acyl-meldrumsäure nach der allgemeinen Formel III. Obwohl hierbei ein zusätzlicher Reaktionsschritt gegenüber der Verfahrensvariante 1 beschritten wird, lassen sich mit dieser Variante verbesserte Ausbeuten und/oder reinere β-Ketoester der allgemeinen Formel II erzielen.

Das erfindungsgemäße strahlungsempfindliche Gemisch kann einen oder mehrere α-Diazo-β-ketoester der allgemeinen Formel I enthalten, insbesondere im gleichen Anteil. Bevorzugt ist allerdings, wenn lediglich eine photoaktive Verbindung im Gemisch enthalten ist. Die Konzentration der α-Diazo-β-ketoester der allgemeinen Formel I im erfindungsgemäßen strahlungsempfindlichen Gemisch kann sich in weiten Grenzen bewegen. Im allgemeinen liegt aber dessen Konzentration im Bereich von 4 bis 40 Gew.-%, insbesondere bei 6 bis 30 Gew.-%, bezogen auf die nichtflüchtigen Bestandteile des strahlungsempfindlichen Gemisches.

Das erfindungsgemäße strahlungsempfindliche Gemisch enthält ferner mindestens ein polymeres, in Wasser unlösliches, in wäßrigen alkalischen Lösungen aber lösliches, zumindest aber quellbares Bindemittel. Das Bindemittel zeichnet sich im besonderen dadurch aus, daß es die Bestandteile des erfindungsgemäßen strahlungsempfindlichen Gemisches gut löst und insbesondere im Wellenlängenbereich von 190 bis 300 nm eine möglichst geringe Eigenabsorption, d.h. eine hohe Transparenz, aufweist. Hierunter fallen insbesondere nicht diejenigen Bindemittel auf der Basis von Novolak-Kondensationsharzen, die in der Regel in Kombination von Naphthochinondiaziden als photoaktive Komponenten eingesetzt worden sind. Zwar lassen Novolak-Kondensationsharze, auch in Kombination mit den genannten α-Diazo-β-ketoestern, nach bildmäßiger Belichtung in den nicht belichteten Bereichen eine Erniedrigung der Löslichkeit gegenüber wäßrig-alkalischen Entwicklern erkennen, doch ist ihre Eigenabsorption in dem für die Belichtung gewünschten Wellenlängenbereich unerwünscht hoch.

Die genannten Novolak-Kondensationsharze können aber in Mischung mit anderen als Bindemittel geeigneten Harzen mit höherer Transparenz eingesetzt werden. Die Mischungsverhältnisse richten sich dabei vorwiegend nach der Art des mit dem Novolakharz zu mischenden Bindemittels. Insbesondere spielen dessen Grad an Eigenabsorption im genannten Wellenlängenbereich, aber auch die Mischbarkeit mit den anderen Bestandteilen des strahlungsempfindlichen Gemisches eine entscheidende Rolle. Im allgemeinen kann aber das Bindemittel des erfindungsgemäßen strahlungsempfindlichen Gemisches bis zu 30 Gew.-%, insbesondere bis zu 20 Gew.-%, eines Novolak-Kondenstationsharzes enthalten.

Als Bindemittel geeignet sind Homo- oder Copolymere des p-Hydroxystyrols sowie seiner Alkylderivate, z.B. des 3-Methylhydroxystyrols, sowie Homo- oder Copolymere anderer Polyvinylphenole, z.B. des 3-Hydroxystyrols oder die Ester oder Amide von Acrylsäure mit phenolischen Gruppen aufweisenden Aromaten. Als Comonomere im Copolymeren können polymerisierbare Verbindungen wie Styrol, Methacrylsäuremethacrylat, Acrylsäuremethacrylat oder ähnliche eingesetzt werden.

Gemische mit erhöhter Plasmabeständigkeit werden dann erhalten, wenn zur Herstellung von Copolymeren des obigen Typs Silizium enthaltende Vinylmonomere, z.B. Vinyltrimethylsilan, verwendet werden. Die Transparenz dieser Bindemittel ist im interessierenden Bereich im allgemeinen höher, so daß eine verbesserte Strukturierung möglich ist.

Mit gleichem Erfolg lassen sich auch Homo- oder Copolymere des Maleinimids verwenden. Auch diese Bindemittel zeigen hohe Transparenz im beschriebenen Wellenlängenbereich. Als Comonomere werden auch hier bevorzugt Styrol, substituierte Styrole, Vinylether, Vinylester, Vinylsilylverbindungen oder (Meth)acrylsäureester eingesetzt.

Schließlich sind darüber hinaus auch Copolymere des Styrols mit Comonomeren verwendbar, die in wäßrig alkalischen Lösungen eine Löslichkeitserhöhung bewirken. Hierunter zählen beispielsweise Maleinsäureanhydrid, Maleinsäurehalbester oder dergleichen.

Die genannten Bindemittel können in Mischungen auftreten, sofern sie mischbar sind und die optischen Qualitäten des strahlungsempfindlichen Gemisches nicht verschlechtern. Bevorzugt sind jedoch Bindemittel, enthaltend einen Typus der oben genannten Arten.

Das erfindungsgemäße strahlungsempfindliche Gemisch enthält 60 bis 96 Gew.-%, insbesondere 70 bis 94 Gew.-% eines Bindemittels, bezogen auf den Anteil der nichtflüchtigen Bestandteile des strahlungsempfindlichen Gemisches.

Ferner können dem erfindungsgemäßen strahlungsempfindlichen Gemisch ggf. Farbstoffe, Pigmente, Netzmittel, Haftvermittler und Verlaufmittel zur Verbesserung spezieller Erfordernisse wie Flexibilität, Haftung und Glanz zugesetzt werden.

Vorzugsweise wird das erfindungsgemäße lichtempfindliche Gemisch in Lösungsmitteln wie Ethylenglykol, Glykolethern wie Glykolmonomethylether, Glykoldimethylether, Glykolmonoethylether oder Propylenglykolmonoalkylethern, insbesondere Propylenglykolmethylether; aliphatischen Estern wie Ethylacetat, Hydroxyethylacetat, Alkoxyethylacetat, γ-Butylacetat, Propylenglykolmonoalkyletheracetat, insbesondere Propylenglykolmethyletheracetat oder Amylacetat; Ethern wie Dioxan, Ketonen wie Methylethylketon, Methyliso-butylketon, Cyclopentanon und Cyclohexanon; Dimethylformamid, Dimethylacetamid, Hexamethylphosphorsäureamid, N-Methylpyrrolidon, Butyrolacton, Tetrahydrofuran, und in Mischungen derselben gelöst. Besonders bevorzugt werden Glykolether, aliphatische Ester sowie Ketone. Darüber hinaus können Lösungsmittelgemische Anwendung finden, die u.a. auch aromatische Kohlenwasserstoffe oder Xylol enthalten. Letztendlich hängt die Wahl der Lösemittel von dem angewandten Beschichtungsverfahren, der gewünschten Schichtstärke und den Trocknungsbedingungen ab. Ebenso müssen die Lösemittel chemisch neutral sein, d.h. sie dürfen nicht mit den übrigen Schichtkomponenten irreversibel reagieren.

Die mit den übrigen Bestandteilen des strahlungsempfindlichen Gemisches entstehenden Lösungen haben in der Regel einen Feststoffgehalt von 5 bis 60 Gew.-%, vorzugsweise bis 50 Gew.-%.

Erfindungsgemäß wird ferner ein strahlungsempfindliches Aufzeichnungsmaterial beansprucht, im wesentlichen bestehend aus einem Substrat und dem darauf aufgetragenen strahlungsempfindlichen Gemisch.

Als Substrate kommen alle Materialien in Frage, aus denen Kondensatoren, Halbleiter, mehrlagige gedruckte Schaltungen oder integrierte Schaltkreise bestehen bzw. hergestellt werden können. Insbesondere sind Oberflächen aus reinen Siliciumschichten und thermisch oxidiertem und/oder mit Aluminium beschichtetem Siliciummaterial zu nennen, die gegebenenfalls auch dotiert sein können, einschließlich aller anderen in der Halbleitertechnologie üblichen Substrate wie beispielsweise Siliciumnitrid, Galliumarsenid, Indiumphosphid. Weiterhin kommen in Frage die aus der Flüssigkristalldisplay-Herstellung bekannten Substrate wie Glas, Indium-Zinnoxid; ferner Metallplatten und -folien, beispielsweise aus Aluminium, Kupfer, Zink; Bimetall- und Trimetallfolien, aber auch elektrisch nicht leitende Folien, die mit Metallen bedampft sind, gegebenenfalls mit Aluminium beschichtete $SiO_2$-Materialien und Papier. Diese Substrate können einer Temperatur-Vorbehandlung unterzogen werden, oberflächlich angerauht, angeätzt oder zur Erzielung erwünschter Eigenschaften, wie z. B. Erhöhung der Hydrophilie, mit Chemikalien behandelt sein.

In einer besonderen Ausführungsform kann das strahlungsempfindliche Gemisch zur besseren Haftung in dem Resist oder zwischen dem Resist und dem Substrat einen Haftvermittler enthalten. Bei Silicium- bzw. Siliciumdioxid-Substraten kommen hierfür Haftvermittler vom Aminosilantyp, wie z.B. 3-Aminopropyltriethoxysilan oder Hexamethyl-disilazan in Frage.

Beispiele für Träger, die zur Herstellung von photomechanischen Aufzeichnungsschichten wie Druckformen für den Hochdruck, den Flachdruck, den Siebdruck, den Tiefdruck sowie von Reliefkopien Verwendung finden können, sind Aluminiumplatten, ggf. anodisch oxidiert, gekörnte und/ oder silikatisierte Aluminiumplatten, Zinkplatten, Stahlplatten, die ggf. mit Chrom bearbeitet wurden, sowie Kunststoffolien oder Papier.

Das erfindungsgemäße Aufzeichnungsmaterial wird bildmäßig belichtet. Quellen aktinischer Strahlung sind: Metallhalogenidlampen, Kohlebogenlampen, Xenonlampen und Quecksilberdampflampen. Ebenso kann eine Belichtung mit energiereicher Strahlung wie Laser-, Elektronen- oder Röntgenstrahlung erfolgen. Besonders bevorzugt sind jedoch Lampen, die Licht einer Wellenlänge von 190 bis 260 nm ausstrahlen können, d.h. insbesondere Xenon- oder/und Quecksilberdampflampen. Darüber hinaus lassen sich auch Laserlichtquellen verwenden, z.B. Excimerlaser, insbesondere KrF- oder ArF-Laser, die bei 249 bzw. 193 nm emittieren. Die Strahlungsquellen müssen in den genannten Wellenlängenbereichen eine ausreichende Emission aufweisen, andererseits aber auch für aktinisches Licht des genannten Bereiches genügend durchlässig sein.

Gegenstand der Erfindung ist ferner ein Verfahren zur Herstellung eines strahlungsempfindlichen Aufzeichnungsmaterials. Das Auftragen des strahlungsempfindlichen Gemisches auf das Substrat kann durch Aufsprühen, Walzen, Rakeln, Gießen, durch Fließbeschichten, Schleuderbeschichten und Tauchbeschichten oder mittels Breitschlitzdüsen erfolgen. Danach wird das Lösungsmittel durch Verdampfen entfernt, so daß auf der Oberfläche des Substrates die strahlungsempfindliche Schicht zurückbleibt. Die Entfernung des Lösemittels kann gegebenenfalls durch Erhitzen der Schicht auf Temperaturen von bis zu 140° C gefördert werden. Das Gemisch kann aber auch zunächst auf obengenannte Weise auf den Zwischenträger aufgetragen werden, von dem aus es unter Druck und erhöhter Temperatur auf das endgültige Trägermaterial übertragen wird.

Die Schichtstärke variiert in Abhängigkeit von ihrem Einsatzgebiet. Sie beträgt zwischen 0,1 und 100 µm, insbesondere zwischen 0,3 und 10 µm.

Anschließend wird die Schicht bildmäßig belichtet. Üblicherweise wird aktinische Strahlung verwendet. Zur Bestrahlung werden bevorzugt UV-Lampen verwendet, die Licht einer Wellenlänge von 190 bis 300 nm aussenden. In der strahlungsempfindlichen Schicht wird anschließend durch Entwicklung ein Bildmuster freigelegt, in dem die Schicht mit einer Entwicklungslösung behandelt wird, die die bestrahlten Bereiche des Materials löst bzw. entfernt.

Als Entwickler werden Lösungen von Reagenzien wie z.B. Silikaten, Metasilikaten, Hydroxiden, Hydrogen- bzw. Dihydrogenphosphaten, Carbonaten bzw. Hydrogencarbonaten, von Alkali und/oder Erdalkali, insbesondere von Ammoniumionen, aber auch Ammoniak und dergleichen verwendet. Metallionenfreie Entwickler werden in der US-A-4,729,941, der EP-A-0 062 733, der US-A-4,628,023, der US-A-4,141,733, der EP-A-0 097 282 und der EP-A-0 023 758 beschrieben. Der Gehalt dieser Substanzen in der Entwicklerlösung beträgt im allgemeinen 0,1 bis 15 Gew.-%, vorzugsweise 0,5 bis 5 Gew.-%, bezogen auf das Gewicht der Entwicklerlösung. Metallionenfreie Entwickler werden insbesondere verwendet. Den Entwicklern können ggf. geringere Mengen eines Netzmittels zugesetzt sein, um die Ablösung der belichteten Stellen im Entwickler zu erleichtern.

Die entwickelten Resiststrukturen werden ggf. nachgehärtet. Dies geschieht im allgemeinen dadurch, daß man die Resiststruktur auf einer hot-plate bis zu einer Temperatur unter der Fließtemperatur erhitzt und anschließend mit UV-Licht einer Xenon-Quecksilber-Dampflampe (Bereich von 200 bis 250 nm) ganzflächig belichtet. Durch diese Nachhärtung werden die Resiststrukturen vernetzt, so daß die Strukturen eine Fließbeständigkeit im allgemeinen bis zu Temperaturen von über 200° C aufweisen. Die Nachhärtung kann auch ohne Temperaturerhöhung unter Einstrahlung von UV-Licht erfolgen. Dies gilt insbesondere dann, wenn energiereiche Strahlung verwendet wird, z.B. Elektronenstrahlung.

Bevorzugte Anwendung findet das erfindungsgemäße strahlungsempfindliche Gemisch in lithographischen Prozessen zur Herstellung integrierter Schaltungen oder von diskreten elektrischen Bausteinen. Das aus dem Gemisch hergestellte Aufzeichnungsmaterial dient dabei als Maske für die folgenden Prozeßschritte. Hierunter zählen z.B. das Ätzen des Schichtträgers, die Implantation von Ionen in den Schichtträger oder die Abscheidung von Metallen oder anderen Materialien auf den Schichtträger.

Die erfindungsgemäßen Gemische zeigen unter den praxisüblichen Bedingungen bei Belichtung im DUV-Bereich gegenüber den bekannten Materialien eine vergleichbare hohe Lichtempfindlichkeit (Vergleichsbeispiel s.u.), wenn sie mit Licht einer Wellenlänge von 190 bis 300 nm bestrahlt werden. Als Vorteile zeigen sie jedoch eine höhere thermische Stabilität als die bekannten Materialien sowie eine hervorragende Kompatibilität.

Die folgenden Beispiele erläutern die Herstellung der α-Diazo-β-ketoester nach der allgemeinen Formel I.

Herstellungsbeispiele:

Beispiel 1

Herstellung eines difunktionellen α-Diazo-β-ketoesters der allgemeinen Formel I: Bis-2,9-diazo-1,10-dicyclohexyl-4,7-dioxa-1,3,8,10-tetraoxo-decan (3)

Stufe 1:

5-(1-Cyclohexyl-1-hydroxy-methyliden)-2,2-dimethyl-1,3-dioxan-4,6-dion (1)

Eine Lösung bestehend aus 144,1 g (1,0 mol) 2,2-Dimethyl-1,3-dioxan-4,6-dion und 158,2 g (2,0 mol) Pyridin in 500 ml Dichlormethan wird auf 10° C gekühlt. Bei dieser Temperatur werden unter Rühren 146,6 g (1,0 mol) Cyclohexancarbonsäurechlorid zugetropft. Dann wird auf Raumtemperatur erwärmt und nach 4 Stunden die Reaktionsmischung mit einer Mischung aus 500 g Eis, 500 ml Wasser und 100 ml konzentrierter Salzsäure versetzt. Die organische Phase wird zweimal mit 400 ml Wasser gewaschen, über Magnesiumsulfat getrocknet und anschließend vom Lösungsmittel befreit.

Es verbleibt ein öliger roter Rückstand, der zweimal mit je 600 ml n-Hexan unter Zusatz von Aktivkohle aufgekocht wird. Aus der n-Hexanlösung fallen beim Erkalten feine, schwach gelb gefärbte Kristalle der gewünschten Verbindung (1) aus. Nach einer weiteren Umkristallisation aus n-Hexan erhält man in einer Ausbeute von 57 % weiße Kristalle der Verbindung (1) mit einem Schmelzpunkt von 82° C.

Stufe 2:

1,10-Dicyclohexyl-4,7-dioxa-1,3,8,10-tetraoxo-decan (2)

12,4 g (0,2 mol) Ethylenglykol und 101,8 g (0,4 mol) der vorstehend beschriebenen Verbindung (1) werden in 800 ml Ethylmethylketon eingetragen und langsam erwärmt. Ab etwa 60° C beginnt eine starke Kohlendioxidentwicklung. Die Lösung wird für 2,5 Stunden am Rückfluß gehalten. Nach Abkühlung der Reaktionsmischung wird das Lösungsmittel im Vakuum abgezogen. Es verbleibt ein öliger Rückstand, der praktisch ausschließlich aus dem gewünschten difunktionellen β-Ketoester (2) besteht. Er wird ohne weitere Reinigung als Ausgangsprodukt für die nächste Stufe verwendet.

Stufe 3:

Bis-2,9-diazo-1,10-dicyclohexyl-4,7-dioxa-1,3,8,10-tetraoxo-decan (3)

11,5 g (30 mmol) des β-Ketoesters (2) werden in 130 ml Acetonitril gelöst und auf 0° C gekühlt. Zu der erkalteten Lösung werden unter Rühren 9,85 g (50 mmol) Tosylazid hinzugefügt und anschließend 6,6 g (65 mmol) Triethylamin derart zugetropft, daß die Temperatur nicht über 5° C steigt. Die Mischung wird 10 Minuten bei dieser Temperatur gerührt; anschließend wird auf Raumtemperatur erwärmt. Nach zwei Stunden ist in der Reaktionsmischung dünnschichtchromatographisch (Kieselgel, Laufmittel: $CH_2Cl_2$) kein Tosylazid mehr nachweisbar. Die Mischung wird unter Kühlung mit 3,4 g (15 mmol) 4-Carboxyphenylsulfonylazid versetzt. Nach

Weiterreaktion für weitere 2 Stunden bei Raumtemperatur bildet sich ein Niederschlag. Die Mischung wird am Rotationsdampfer eingeengt, in Diethylether aufgenommen, zweimal mit je 5%iger wässriger Kalilauge extrahiert und mit Wasser neutral gewaschen. Die organische Phase wird abgetrennt und über Magnesiumsulfat getrocknet. Nach deren Einengen verbleiben 12,2 g eines schwach gelblichen Öls, das durch Anlegen eines Hochvakuums von Lösungsmittelresten befreit wird. Nach einer Chromatographie an Kieselgel mit Dichlormethan als Laufmittel erhält man das farblose Produkt (3), welches folgende Zusammensetzung aufweist.

| | | | | |
|---|---|---|---|---|
| $C_{22}H_{18}N_4O_6$ | ber.: | C 60,8 % | H 4,2 % | N 12,9 % |
| (MG 434.41) | gef.: | C 60,7 % | H 4,3 % | N 13,0 % |

IR (Film): 2.140,7 $cm^{-1}$ ($C=N_2$)

Beispiel 2

Herstellung eines ungesättigten, difunktionellen α-Diazo-β-ketoesters der allgemeinen Formel I: Bis-4,13-diazo-6,11-dioxa-2,2,15,15-tetramethyl-3,5,12,14-tetraoxo-hexadec-8-in (5)

Stufe 1:

6,11-Dioxa-2,2-15,15-tetramethyl-3,5,12,14-tetraoxohexa-dec-8-in (4)

8,6 g (0,1 mol) 2-Butin-1,4-diol werden unter Rühren zusammen mit 47,5 g (0,3 mol) 4,4-Dimethyl-3-oxovaleriansäuremethylester auf 120° C erwärmt. Der dabei entstehende Methylalkohol wird in eine gekühlte Vorlage abdestilliert. Nach etwa 4 Stunden ist die theoretisch berechnete Menge an Methylalkohol abdestilliert. Die homogene Lösung wird 1 Stunde auf 140° C erhitzt und überschüssige 4,4-Dimethyl-3-oxovaleriansäure unter Anlegen eines Vakuums (10 Torr) abdestilliert. Der verbleibende Rückstand entspricht praktisch der reinen Verbindung (4) und wird ohne weitere Reinigung weiter verarbeitet.

Stufe 2:

Bis-4,13-diazo-6,11-dioxa-2,2,15,15-tetramethyl-3,5,12,14-tetraoxo-hexadec-8-in (5)

30,4 g (90 mmol) des vorstehend beschriebenen Produkts (4) werden in 250 ml Acetonitril gelöst und auf 0° C abgekühlt. Unter Rühren werden portionsweise 33,6 g (170 mmol) Tosylazid derart zugetropft, daß die Temperatur 5° C nicht übersteigt. Die Mischung wird auf Raumtemperatur erwärmt. Nach etwa 2 Stunden kann dünnschichtchromatographisch kein Tosylazid mehr nachgewiesen werden. Die Mischung wird mit 4,5 g (20 mmol) 4-Carboxybenzolsulfonylazid versetzt. Nach zwei Stunden wird das Lösungsmittel am Rotationsverdampfer abdestilliert, der feste Rückstand in Dichlormethan aufgenommen und mit 2x 200 ml 4%iger Kalilauge, sowie anschließend mit 200 ml Wasser gewaschen. Nach Trocknen und Entfernung des Lösungsmittels verbleibt ein Öl, welches im Kühlschrank zu kristallisieren beginnt. Der Kristallkuchen (29,8 g = 85 % d. Th.) wird aus n-Hexan umkristallisiert. Man erhält 22,2 g farblose Kristalle der Verbindung (5) vom Schmelzpunkt 69 - 70° C.

| | | | | |
|---|---|---|---|---|
| $C_{18}H_{22}N_4O_6$ | ber.: | C 55,38 | H 5,68 | N 14,35 |
| (MG 390,40) | gef.: | C 55,4 | H 5,6 | N 14,4 |

IR (KBR): 2.137,9 $cm^{-1}$, 2.160,0 $cm^{-1}$ (Schulter) ($C=N_2$).

Beispiel 3

Herstellung eines trifunktionellen α-Diazo-β-ketoesters der allgemeinen Formel I: Tris-[(5-diazo-3,8-dioxa-4,6-dioxo-8-phenyl)-octyl]-amin (8)

Stufe 1:

5-(1-Hydroxy-2-phenoxy-ethyliden)-2,2-dimethyl-1,3-dioxan-4,6-dion (6)

In eine auf 8° C gekühlte Lösung aus 72,1 g (0,5 mol) 2,2-Dimethyl-1,3-dioxan-4,6-dion und 79,1 (1,0 mol)

Pyridin in 250 ml Dichlormethan werden 93,8 g (0,55 mol) Phenoxyessigsäurechlorid derart zugetropft, daß die Temperatur nicht über 10° C ansteigt. Die Mischung wird 2,5 Stunden bei Raumtempertur nachgerührt, mit einer Mischung aus 250 g Eis, 250 ml Wasser und 30 ml konzentrierter Salzsäure versetzt und ausgeschüttelt. Die organische Phase wird zweimal mit je 100 ml Wasser gewaschen und über Magnesiumsulfat getrocknet. Das Lösungsmittel wird abdestilliert, wobei ein Feststoff verbleibt, der aus t-Butylmethylether umkristallisiert wird. Man erhält 72 g (52 % d. Th.) weißer Kristalle der Verbindung (6), die bei 85 - 87° C unter Zersetzung schmelzen.

Stufe 2:

Tris-[(3,8-dioxa-4,6-dioxo-8-phenyl)-octyl]-amin (7)

3,6 g (20 mmol) Triethanolamin und 18 g (64 mmol) der vorstehend beschriebenen Verbindung (6) werden in 140 ml Methylethylketon zum Rückfluß erhitzt. Nachdem die Kohlendioxidentwicklung beendet ist, wird eine weitere Stunde zum Rückfluß erhitzt, und anschließend das Lösungsmittel abdestilliert. Es verbleibt hochviskoses, rötliches Öl der Verbindung (7), das ohne weitergehende Reinigung für die nachfolgende Reaktionsstufe eingesetzt wird.

Stufe 3:

Tris-[(5-diazo-3,8-dioxa-4,6-dioxo-8-phenyl)-octyl]-amin (8)

Die Gesamtmenge des in Stufe 2 entstandenen Öls (7) (etwa 20 mmol) wird in 120 mol Acetonitril gelöst und auf 0° C gekühlt. Zu der erkalteten Lösung werden unter Rühren 6,5 g (65 mmol) Triethylamin hinzugefügt und anschließend 12,8 g (65 mmol) Tosylazid in der Weise zugetropft, daß die Temperatur nicht über 5° C ansteigt. Die Mischung wird 10 Minuten bei dieser Temperatur gerührt und anschließend auf Raumtemperatur erwärmt. Nach drei Stunden wird die klare Lösung am Rotationsverdampfer zum Trocknen eingeengt, in Diethylether aufgenommen und zweimal mit je 100 ml 5%iger wässriger Kalilauge extrahiert sowie anschließend mit Wasser neutral gewaschen. Die organische Phase wird abgetrennt und über Magnesiumsulfat getrocknet. Nach dem Einengen am Rotationsverdampfer verbleiben 15,4 g eines schwach bräunlichen Öls, das durch Anlegen eines Hochvakuums von Lösungsmittelresten befreit wird. Dabei beginnen sich Kristalle abzuscheiden. Der Kristallbrei wird aus Toluol umkristallisiert, wobei 12,1 g farbloser Kristalle der Verbindung (8) entstehen, die unter beginnender Zersetzung bei 104° C schmelzen.

| | | | | |
|---|---|---|---|---|
| $C_{36}H_{33}N_7O_{12}$ | ber.: | C 61,10 % | H 4,70 % | N 13,85 % |
| (MG 707,70) | gef.: | C 61,0 % | H 4,7 % | N 14,1 % |

IR (KBr): 2.142 $cm^{-1}$ ($C=N_2$)

Beispiel 4

Herstellung eines trifunktionellen α-Diazo-β-ketoesters der allgemeinen Formel I: Tris-[(5-diazo-7,7-dimethyl-4,6-dioxo-3-oxa)-octyl]-amin (10)

Stufe 1:

Tris-[(7,7-dimethyl-4,6-dioxo-3-oxa)-octyl]-amin (9)

7,5 g (50 mmol) Triethanolamin und 30 g (189 mmol) 4,4-Dimethyl-3-oxovaleriansäuremethylester werden unter Rühren 5 Stunden auf 130° C erhitzt. Der dabei gebildete Methylalkohol wird abdestilliert. Anschließend wird der überschüssige monofunktionelle β-Ketoester im Vakuum abdestilliert. Zurück bleibt ein hellgelbes Öl, welches sich als fast reine Verbindung (9) erweist, so daß es ohne weitere Reinigung für die nachfolgende Reaktionsstufe verwendet werden kann.

Stufe 2:

Tris-[(5-diazo-7,7-dimethyl-4,6-dioxo-3-oxa)-octyl]-amin (10)

18 g (34 mmol) der Verbindung (9) aus Stufe 1 werden in 180 ml Acetonitril gelöst und auf 0° C gekühlt. Zu der erkalteten Lösung werden unter Rühren 11,4 g (112 mmol) Triethylamin hinzugefügt und anschließend

22,4 g (112 mmol) Tosylazid derart zugetropft, daß die Temperatur nicht über 5° C ansteigt. Die Mischung wird 10 Minuten bei dieser Temperatur gerührt und anschließend auf Raumtemperatur erwärmt. Nach zwei Stunden wird die klare Lösung am Rotationsverdampfer zur Trockne eingeengt, in Diethylether aufgenommen, zweimal mit je 100 ml 5%iger wässriger Kalilauge extrahiert und mit Wasser neutral gewaschen. Die organische Phase wird abgetrennt und über Magnesiumsulfat getrocknet. Nach dem Einengen am Rotationsverdampfer verbleiben 18,4 g eines schwach bräunlichen Öls, daß durch Anlegen eines Hochvakuums von Lösungsmittelresten befreit wird. Dabei beginnen sich Kristalle abzuscheiden. Der Kristallbrei wird aus Cyclohexan umkristallisiert, wobei 15,4 g farbloser Kristalle der Verbindung (10) anfallen, die unter langsamer Zersetzung bei 101° C schmelzen.

| | | | | |
|---|---|---|---|---|
| $C_{27}H_{39}N_7O_9$ | ber.: | C 53,55 % | H 6,49 % | N 16,19 % |
| (MG 605.65) | gef.: | C 53,5 % | H 6,7 % | N 16,1 % |

IR (KBr): 2.171 $cm^{-1}$, 2.134 $cm^{-1}$ (Schulter) ($C=N_2$)

Beispiel 5

Herstellung eines trifunktionellen α-Diazo-β-ketoesters der allgemeinen Formel I: N-Phenyl-N'-[1,1-bis-(5-cyclohexyl-4-diazo-3,5-dioxo-2-oxa-pentyl)-6-cyclohexyl-5-diazo-4,6-dioxo-3-oxa-hexyl]-harnstoff (13)

Stufe 1:

N-Phenyl-N'-[1,1-bis-hydroxymethyl-2-hydroxyethyl]-harnstoff (11)

66,6 g (0,55 mol) Tris(hydroxymethyl)aminomethan werden in 200 ml destilliertem Wasser gelöst und mit 400 ml Aceton versetzt. Unter Rühren werden bei 20° C 59,6 g (0,5 mol) Phenylisocyanat zugetropft, wobei sich ein schwerer, weißer Niederschlag bildet. Nach Beendigung der Zugabe wird weitere 2 Stunden gerührt und der Niederschlag abgesaugt. Er wird anschließend nacheinander in je 150 ml Wasser, Aceton und Diethylether digeriert. Schließlich wird die praktisch analysenrein erhaltene Verbindung (11) bei 75° C getrocknet.

Stufe 2:

N-Phenyl-N'-[1,1-bis-(5-cyclohexyl-3,5-dioxo-2-oxa-pentyl)-6-cyclohexyl-4,6-dioxo-3-oxa-hexyl]-harnstoff (12)

8,4 g (35 mmol) der Verbindung (11) und 22,0 g (119 mmol) 3-Cyclohexyl-3-oxo-propionsäuremethylester werden in einer Claisenapparatur unter Anlegen eines schwachen Vakuums 4 Stunden auf 130° C erwärmt, wobei der gebildete Methylalkohol abdestilliert. Anschließend wird der überschüssige 3-Cyclohexyl-3-oxo-propionsäuremethylester (Kp. 75 - 76° C/0,05 Torr) aus dem Gemisch abdestilliert. Es verbleibt ein bräunliches, hochviskoses Öl von N-Phenyl-N'- 1,1-bis-(5-cyclohexyl-3,5-dioxo-2-oxa-pentyl)-6-cyclohexyl-4,6-dioxo-3-oxa-hexyl-harnstoff (12), das ohne weitere Reinigung weiter verwendet wird.

Stufe 3:

N-Phenyl-N'-[1,1-bis-(5-cyclohexyl-4-diazo-3,5-dioxo-2-oxa-pentyl-6-cyclohexyl-5-diazo-4,6-dioxo-3-oxahexyl]-harnstoff (13)

13,72 g (20 mmol) der vorstehend beschriebenen Verbindung (12) und 6,6 g (65 mmol) Triethylamin werden in 140 ml Acetonitril gelöst und auf 0° C gekühlt. Zu dieser Lösung werden 12,8 g (65 mmol) Toluolsulfonylazid derart zugetropft, daß die Temperatur 5° C nicht übersteigt. Die Mischung wird auf Raumtemperatur erwärmt und 4 Stunden nachgerührt. Anschließend wird die klare Lösung am Rotationsverdampfer zur Trockne eingeengt, der Rückstand in Dichlormethan aufgenommen, zweimal mit je 100 ml 5%-iger wäßriger Kalilauge extrahiert und mit Wasser neutral gewaschen. Die organische Phase wird abgetrennt und über Magnesiumsulfat getrocknet. Nach dem Einengen am Rotationsverdampfer verbleiben 12,4 g eines schwach bräunlichen Öls, das durch Anlegen eines Hochvakuums von Lösungsmittelresten befreit wird. Das Öl wird erneut in einer geringen Menge Dichlormethan aufgenommen und über eine Kieselgelsäule eluiert. Durch Verwendung von Dichlormethan/Essigester 5:1 als mobile Phase werden, nach Einengen des Lösungsmittels, 8,5 g (55,6 % d. Th.) eines schwach gelblichen Öls der Verbindung (13) erhalten.

| | | | | |
|---|---|---|---|---|
| $C_{38}H_{48}N_8O_{10}$ | ber.: | C 58,91 % | H 5,98 % | N 14,46 % |
| (MG 764,76) | gef.: | C 59,2 % | H 6,0 % | N 14,5 % |

IR (KBr): 2.141,2 $cm^{-1}$ ($C=N_2$)

Beispiel 6

Herstellung eines trifunktionellen α-Diazo-β-ketoesters der allgemeinen Formel I: 2,2,2-Tris-[4-(7-cyclohexyl-6-diazo-1,4-dioxa-5,7-dioxo-heptyl)-phenyl]-ethan (15)

Stufe 1:

2,2,2-Tris-[4-(7-cyclohexyl-1,4-dioxa-5,7-dioxoheptyl)-phenyl]-ethan (14)

8,77 g (20 mmol) 2,2,2-Tris-[4-(2-hydroxy-ethoxy)-phenyl]-ethan (hergestellt durch Umsetzung von Tris-(4-hydroxyphenyl)-ethan mit 2-Chlorethanol) werden mit 18,4 g (100 mmol) 3-Cyclohexyl-3-oxopropionsäuremethylester in einer Claisenapparatur unter Anlegen eines schwachen Vakuums 4 Stunden auf 130° C erwärmt. Der gebildete Methylalkohol wird abdestilliert, ebenso der überschüssige 3-Cyclohexyl-3-oxo-propionsäuremethylester (Kp. 75 - 76° C/0,05 Torr). Es verbleibt ein schwach bräunlich gefärbtes hochviskoses Öl der Verbindung (14), das ohne weitere Reinigung weiter verwendet wird.

Stufe 2:

2,2,2-Tris-[4-(7-cyclohexyl-6-diazo-1,4-dioxa-5,7-dioxo-heptyl)-phenyl]-ethan (15)

8,95 g (10 mmol) der vorstehend beschriebenen Verbindung (14) und 3,5 g (35 mmol) Triethylamin werden in 100 ml Acetonitril gelöst und auf 0° C abgekühlt. Zu dieser Lösung werden 6,9 g (35 mmol) Toluolsulfonylazid derart zugetropft, daß die Temperatur 5° C nicht übersteigt. Die Mischung wird auf Raumtemperatur erwärmt und 4 Stunden nachgerührt. Anschließend wird die klare Lösung am Rotationsverdampfer zur Trockne eingeengt, in Dichlormethan aufgenommen, zweimal mit je 100 ml 5%-iger wäßriger Kalilauge extrahiert und mit Wasser neutral gewaschen. Die organische Phase wird abgetrennt und über Magnesiumsulfat getrocknet. Nach dem Einengen am Rotationsverdampfer verbleiben 9,4 g eines schwach bräunlichen Öls, das durch Anlegen eines Hochvakuums von Lösungsmittelresten befreit wird. Das Öl beginnt unmittelbar zu kristallisieren. Der Kristallbrei wird aus t-Butylmethylether umkristallisiert. Die Verbindung (15) wird in Form eines weißen Pulvers in praktisch quantitiver Ausbeute erhalten und besitzt einen Schmelzpunkt von 96° C.

| | | | | |
|---|---|---|---|---|
| $C_{53}H_{60}N_6O_{12}$ | ber.: | C 65,42 % | H 6,22 % | N 8,64 % |
| (MG 973,09) | gef.: | C 65,4 % | H 6,3 % | N 8,4 % |

IR (KBr): 2.141,2 $cm^{-1}$ ($C=N_2$)

Beispiel 7

Herstellung eines tetrafunktionellen α-Diazo-β-ketoesters der allgemeinen Formel I: N-1,1-Bis-(5-cyclohexyl-4-diazo-3,5-dioxo-2-oxa-pentyl)-6-cyclohexyl-5-diazo-4,6-dioxo-3-oxa-hexyl]-O-[6-cyclohexyl-5-diazo-4,6-dioxo-3-oxa-hexyl)-urethan (18)

Stufe 1:

N-(Bis-1,1-hydroxymethyl-2-hydroxyethyl)-O-(2-hydroxyethyl)-urethan (16)

88,6 g (1 mol) Ethylencarbonat und 121,2 g Tris-(hydroxymethyl)-aminomethan werden miteinander vermischt. Die Mischung erhitzt sich und wird durch externe Kühlung auf maximal 70° C gehalten. Nachdem die exotherme Reaktion beendet ist, wird weitere 6 Stunden bei dieser Temperatur gerührt. Beim Abkühlen bildet sich eine Schmelze, die aus Aceton umkristallisiert wird. Man erhält 198 g weißer Kristalle, die bei 112° C schmelzen.

Stufe 2:

N-[1,1-Bis-(5-cyclohexyl-3,5-dioxo-2-oxa-pentyl)-6-cyclohexyl-4,6-dioxo-3-oxa-hexyl]-O-[6-cyclohexyl-4,6-dioxo-3-oxa-hexyl)-urethan (17)

6,27 g (30 mmol) der Verbindung (16) aus Stufe 1 werden mit 25,0 g (136 mmol) Cyclohexylacetessigsäuremethylester auf 140° C erhitzt. Im Verlauf von 4 Stunden wird die theoretische Menge von Methylalkohol aus dem Gemisch abdestilliert. Anschließend wird der Überschuß des Cyclohexylacetessigsäuremethylesters im Vakuum abdestilliert, und der schwach bräunliche Rückstand der Verbindung (17) ohne weitere Reinigung weiterverarbeitet.

Stufe 3:

N-[1,1-Bis-(5-cyclohexyl-4-diazo-3,5-dioxo-2-oxa-pentyl)-6-cyclohexyl-5-diazo-4,6-dioxo-3-oxa-hexyl]-O-[6-cylcohexyl-5-diazo-4,6-dioxo-3-oxa-hexyl)-urethan (18)

24,5 g (30 mmol) der Verbindung (17) werden in 250 ml Acetonitril gelöst und auf 0° C gekühlt. Zu der erkalteten Lösung werden unter Rühren 13,4 g (130 mmol) Triethylamin hinzugefügt. Anschließend werden 25,6 g (130 mmol) Tosylazid derart zugetropft, daß die Temperatur nicht über 5° C ansteigt. Die Mischung wird 10 Minuten bei dieser Temperatur gerührt und anschließend auf Raumtemperatur erwärmt. Nach 3 Stunden wird die klare, rötlich-braune Lösung am Rotationsverdampfer zur Trockne eingeengt, der Rückstand in Dichlormethan aufgenommen, zweimal mit je 100 ml 5%iger wässriger Kalilauge extrahiert und mit 5%iger wässriger Kochsalz-Lösung neutral gewaschen. Die organische Phase wird anschließend abgetrennt und über Magnesiumsulfat getrocknet. Nach Einengen der Lösung am Rotationsverdampfer verbleiben 26,7 g eines bräunlichen Öls, das durch Anlegen eines Hochvakuums von Lösungsmittelresten befreit wird. Durch Zugabe von Diethylether beginnen sich geringe Mengen schwach gelblicher Kristalle abzuscheiden. Diese werden abgetrennt und erweisen sich als N-[1,1-Bis-(5-cyclohexyl-4-diazo-3,5-dioxo-2-oxa-pentyl)-6-cyclohexyl-5-diazo-4,6-dioxo-3-oxa-hexyl]-O-[6-cyclohexyl-5-diazo-4,6-dioxo-3-oxa-hexyl)-urethan (18). Die verbleibende Lösung wird an Kieselgel mit Dichlormethan chromatographiert, um restliche Spuren an Verbindung (18) zu isolieren. Überschüssiges Tosylazid wird dabei eluiert. Die Hauptfraktion wird mit einer Mischung aus Dichlormethan/Essigester eluiert (75 % der eingesetzten Menge), wobei nach Abdestillierung der Lösemittel die Kristallisation von Verbindung (18) einsetzt.

| | | | | |
|---|---|---|---|---|
| $C_{43}H_{55}N_9O_{14}$ | ber.: | C 56,02 % | H 6,01 % | N 13,62 % |
| (MG 921,96) | gef.: | C 56,1 % | H 5,8 % | N 13,4 % |

IR (KBr): 2.140,6 $cm^{-1}$ ($C=N_2$)

Beispiel 8

Herstellung eines tetrafunktionellen α-Diazo-β-ketoesters der allgemeinen Formel 1: N,N,N',N'-Tetrakis-[6-cyclohexyl-5-diazo-4,6-dioxo-3-oxa-hexyl]-ethylendiamin (20)

Stufe 1:

N,N,N',N'-Tetrakis- 6-cyclohexyl-4,6-dioxo-3-oxahexyl -ethylendiamin (19)

5,9 g (25 mmol) N,N,N',N'-Tetrakis-(2-hydroxyethyl)-ethylendiamin werden mit 33,1 g (180 mmol) 3-Cyclohexyl-3-oxo-propionsäuremethylester 7,5 Stunden auf 130° C erhitzt, wobei der gebildete Methylalkohol abdestilliert wird. Anschließend wird der Überschuß des 3-Cyclohexyl-3-oxo-propionsäuremethylesters in Vakuum abdestilliert. Man erhält in quantitativer Ausbeute ein orangegelbes zähes Öl der sehr reinen Verbindung (19). Dieses wird ohne weitere Reinigung in der nächsten Reaktionsstufe eingesetzt.

Stufe 2:

N,N,N',N'-Tetrakis-[6-cyclohexyl-5-diazo-4,6-dioxo-3-oxa-hexyl]-ethylendiamin (20)

19,8 g (23 mmol) der vorstehend beschriebenen Verbindung werden in 200 ml Acetonitril gelöst. In die auf 0° C gekühlte Lösung werden 20,6 g (104 mmol) Tosylazid gegeben und anschließend 10,8 g (107 mmol) Triethylamin derartig zugetropft, daß die Temperatur nicht über 50° C ansteigt. Nach drei Stunden wird die klare,

rötliche Lösung am Rotationsverdampfer zur Trockne eingeengt, in einer Ether/Dichlormethan-(3:1)-Mischung aufgenommen, zweimal mit je 100 ml 5%-iger wäßriger Kalilauge extrahiert und mit 5%-iger wäßriger Kochsalzlösung neutral gewaschen. Die organische Phase wird abgetrennt und über Magnesiumsulfat getrocknet. Bei dem Einengen am Rotationsverdampfer fällt das fast reine Produkt (20) bereits kristallin und in 95%-iger Ausbeute an. Eine analysenreine Probe wird durch Umkristallisation aus Ethanol hergestellt; weiße Kristalle mit einem Schmelzpunkt von 112 - 114° C.

| | | | | |
|---|---|---|---|---|
| $C_{46}G_{64}N_{10}O_{12}$ | ber.: | C 58,22 % | H 6,80 % | N 14,76 % |
| (MG 949,07) | gef.: | C 58,1 % | H 7,0 % | N 14,5 % |

IR (KBr): 2.140,6 $cm^{-1}$ ($C{=}N_2$)

Beispiel 9

Herstellung eines hexafunktionellen α-Diazo-β-ketoesters der allgemeinen Formel I: 1,19-Bis-cyclohexyl-2,18-bis-diazo-6,14-[bis-(4-cyclohexyl-3-diazo-2,4-dioxo-1-oxa)-butyl]-10-[8-(cyclohexyl-7-diazo-1,5-dioxa-6,8-dioxo-3-(3-cyclohexyl-3-diazo-2,4-dioxo-1-oxabutyl))-1,5-dioxa-6,8-dioxo-octyl]-4,8,12,16-tetraoxa1,3,17,19-tetraoxo-nonadecan (22)

Stufe 1:

1,19-Bis-cyclohexyl-6,14-[bis-(4-cyclohexyl-2,4-dioxo-1-oxa)-butyl]-10-[8-(cyclohexyl-1,5-dioxa-6,8-dioxo-3-(3-cyclohexyl-2,4-dioxo-1-oxa-butyl))-1,5-dioxa-6,8-dioxo-octyl]-4,8,12,16-tetraoxa-1,3,17,19-tetraoxo-nonadecan (21)

6,32 g (20 mmol) Tetraglycerin werden mit 33,1 g (180 mmol) 3-Cyclohexyl-3-oxo-propionsäuremethylester 7,5 Stunden auf 130° C erhitzt, wobei der gebildete Methylalkohol abdestilliert wird. Anschließend wird der Überschuß des 3-Cyclohexyl-3-oxo-propionsäuremethylesters im Vakuum abdestilliert. Man erhält in quantitativer Ausbeute ein praktisch farbloses glasartiges Produkt aus sehr reinem 1,19-Bis-cyclohexyl-6,14-[bis-(4-cyclohexyl-2,4-dioxo-1-oxa)-butyl]-10-[8-(cyclohexyl-1,5-dioxa-6,8-dioxo-3-(3-cyclohexyl-2,4-dioxo-1-oxa-butyl))-1,5-dioxa-6,8-dioxo-octyl-]-4,8,12,16-tetraoxa-1,3,17,19-tetraoxo-nonadecan (21). Dieses wird ohne weitere Reinigung in der nächsten Reaktionsstufe eingesetzt.

Stufe 2:

1,19-Bis-cyclohexyl-2,18-bis-diazo-6,14-[bis-(3-diazo-4-cyclohexyl-2,4-dioxo-1-oxa)-butyl]-10-[8-(cyclohexyl-7-diazo-1,5-dioxa-6,8-dioxo-3-(3-cyclohexyl-3-diazo-2,4-dioxo-1-oxa-butyl))-1,5-dioxa-6,8-dioxo-octyl]-4,8,12,16-tetraoxa-1,3,17,19-tetraoxo-nonadecan (22)

12,3 g (10 mmol) der Verbindung (21) werden in 150 ml Acetonitril gelöst und wie vorstehend beschrieben mit 11,8 g (60 mmol) Tosylazid und 6,1 g (60 mmol) Triethylamin versetzt. Nach Erwärmung auf Raumtemperatur wird die Mischung 8 Stunden gerührt und wie vorstehend beschrieben aufgearbeitet. Die Hälfte des Produktgemischs wird über eine Kieselgelsäule eluiert, wobei eine hellgelbe Fraktion unter Verwendung des Laufmittels Dichlormethan/Essigester (6:1) isoliert wird. Nach Einengen verbleibt ein gelbliches Öl.

| | | | | |
|---|---|---|---|---|
| $C_{66}H_{86}N_{12}O_{21}$ | ber.: | C 57,30 % | H 6,27 % | N 12,15 % |
| (MG 1383,48) | gef.: | C 56,7 % | H 6,5 % | N 11,9 % |

IR (Film): 2.138,4 $cm^{-1}$ ($C{=}N_2$)

Beispiel 10

Wie in Beispiel 7 beschrieben, werden 10 g (73,5 mmol) Pentaerythrit mit 55,2 g 3-Cyclohexyl-3-oxo-propionsäuremethylester umgesetzt und aufgearbeitet. Es kann ein tetrafunktioneller Ester (Verbindung 23), in dem $R^1 = C_6H_{11}$ bedeutet, isoliert werden. Er fällt als zähes Öl in nahezu quantitativer Ausbeute an.

Anschließend wird der Ester (23) mit 59,1 g (300 mmol) Toluolsulfonylazid und 30,6 g (300 mmol) Triethylamin in 200,0 g Acetonnitril zum entsprechenden tetrafunktionellen α-Diazo-β-ketoester der allgemeinen

Formel I umgesetzt. Die Verbindung (25) wird durch Umkristallisation aus Ethanol gereinigt. Sie zeigt einen Schmelzpunkt von 123 - 125° C.

| | | | | |
|---|---|---|---|---|
| $C_{41}H_{52}N_8O_{12}$ | ber.: | C 58,01 % | H 6,17 % | N 13,20 % |
| (MG 848,91) | gef.: | C 58,2 % | H 6,2 % | N 13,2 % |

Beispiel 11 (Vergleichsbeispiel)

Wie in Beispiel 10 beschrieben, werden 10 g Pentaerythrit zu einem tetrafunktionellen Ester (Verbindung 24) umgesetzt, in diesem Fall aber mit 39,0 g Acetessigsäuremethylester, so daß $R^1 = CH_3$ bedeutet.

Anschließend wird die Verbindung (24), wie in Beispiel 10 beschrieben, zu dem entsprechenden tetrafunktionellen α-Diazo-β-ketoester (26) der allgemeinen Formel I umgesetzt, und die Verbindung (26) aus Ethanol umkristallisiert. Sie zeigt einen Schmelzpunkt von 108 - 120° C.

| | | | | |
|---|---|---|---|---|
| $C_{21}H_{20}N_8O_{12}$ | ber.: | C 43,76 % | H 3,50 % | N 19,44 % |
| (MG 576,43) | gef.: | C 43,9 % | H 3,6 % | N 19,5 % |

Wie in der gleichzeitig eingereichten deutschen Patentanmeldung P 3900735.9 (Hoe 89/K 002) gezeigt wird, zeigen lichtempfindliche Gemische, die die Verbindung gemäß dem Beispiel 11 als photoaktive Komponente enthalten, im Vergleich zu der erfindungsgemäßen Verbindung der allgemeinen Formel I zwar vergleichbare Ausbleicheigenschaften mit diesen, allerdings sind die bilddifferenzierenden Eigenschaften nicht zufriedenstellend.

Beispiele 12 bis 67

Es werden weitere Verbindungen der allgemeinen Formel I vorgestellt, die analog zu den bisher beschriebenen Beispielen hergestellt wurden. Wegen der Vielzahl der Verbindungen werden diese in den folgenden Tabellen hinsichtlich der in der allgemeinen Formel I beschriebenen Variationsmöglichkeiten charakterisiert. Als Analysenwert ist die quantitative Bestimmung des Stickstoffs hinreichend aussagefähig.

| Nr. | $R^1$ | m | Y | n | ber./gef. N% |
|---|---|---|---|---|---|
| 12 | $C_6H_5-CH_2-$ | 2 | $-C_2H_4-$ | 0 | 12,90 / 13,0 |
| 13 | $CH_3O-C(O)-C_2H_4-$ | 2 | " | 0 | 12,22 / 12,5 |
| 14 | $CH_3-C(O)-C_2H_4-$ | 2 | " | 0 | 19,80 / 20,1 |
| 15 | $C_6H_5-CH_2-O-CH_2$ | 2 | " | 0 | 11,24 / 11,2 |
| 16 | $n-C_4H_9$ | 2 | $-CH_2CH_2CH_2-$ | 0 | 14,73 / 14,8 |
| 17 | 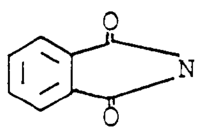 $-CH_2-$ | 2 | $-CH_2-CH-CH_3$ | 0 | 14,33 / 14,2 |
| 18 | $i-C_4H_9-$ | 2 | $-CH_2-CH-CH_2CH_3$ | 0 | 14,20 / 14,3 |
| 19 | $n-C_{14}H_{29}-$ | 2 | $CH_3-CH-CH-CH_3$ | 0 | 8,30 / 8,3 |
| 20 | $n-C_6H_{13}-$ | 2 | $-C_2H_4-O-C_2H_4-$ | 0 | 12,44 / 12,6 |
| 21 | $t-C_4H_9-$ | 2 | $-C_2H_4-S-C_2H_4-$ | 0 | 13,14 / 13,1 |
| 22 | $C_6H_{11}-$ | 2 | $CH_3-N(C_2H_4)_2-$ | 0 | 15,51 / 15,3 |
| 23 | $C_6H_5-O-CH_2-$ | 2 | $-CH_2-(CH_2)_4-CH_2$ | 0 | 10,72 / 10,8 |

| Nr. | $R^1$ | m | X | n | ber./gef. N% |
|---|---|---|---|---|---|
| 24 | $n-C_4H_9-$ | 2 | $-CH_2-CH=CH-CH_2-$ | 0 | 14,28 / 14,1 |
| 25 | $C_6H_{11}-$ | 2 | $-CH_2-C≡C-CH_2-$ | 0 | 12,66 / 12,6 |
| 26 | $C_6H_5-CH_2-$ | 2 | " | 0 | 12,22 / 12,5 |
| 27 | $n-C_8H_{17}-$ | 2 | " | 0 | 11,15 / 11,2 |
| 28 | $CH_3OC(O)-C_2H_4-$ | 2 | " | 0 | 11,22 / 11,2 |
| 29 | $C_6H_{11}-$ | 2 | $-C_2H_4-O-\overset{O}{C}-NH-C_2H_4-NH-\overset{O}{C}-O-C_2H_4-$ | 0 | 14,18 / 13,9 |
| 30 | $CH_3OC_2H_4OCH_2-$ | 2 | 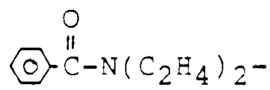 | 0 | 12,84 / 12,5 |
| 31 | 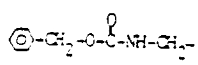 | 2 | 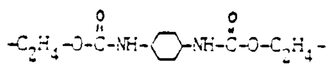 | 0 | 13,89 / 14,1 |
| 32 | $C_6H_{11}-$ | 2 | 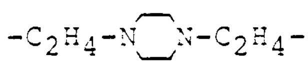 | 0 | 15,84 / 16,0 |
| 33 | $C_6H_5-CH_2-$ | 2 | " | 0 | 15,38 / 15,8 |
| 34 | $C_6H_{11}-$ | 2 | 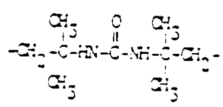 | 0 | 15,94 / 16,2 |
| 35 | $t-C_4H_9-$ | 3 | $-CH_2-\overset{\mid}{C}H-CH_2-$ | 0 | 15,32 / 15,3 |
| 36 | $C_6H_{11}-$ | 3 | " | 0 | 13,41 / 13,2 |
| 37 | " | 3 | " | 1 | 12,49 / 12,2 |

| Nr. | $R^1$ | m | X | n | ber./gef. N% |
|---|---|---|---|---|---|
| 38 | $C_6H_{11}-$ | 3 | $N(C_2H_4)_3-$ | 0 | 14,34 / 14,5 |
| 39 | $CH_3-(CH_2)_7-$ | 3 | " | 0 | 12,67 / 12,4 |
| 40 | $C_6H_5-CH_2-$ | 3 | " | 0 | 13,85 / 13,9 |
| 41 | $CH_3-(CH_2)_{16}-$ | 3 | " | 0 | 8,51 / 8,2 |
| 42 | $H_5C_2O-\overset{O}{\overset{\Vert}{C}}-(CH_2)_3-$ | 3 | " | 0 | 12,57 / 12,3 |
| 43 | $t-C_4H_9-$ | 3 | 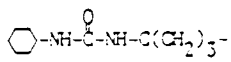 | 0 | 15,94 / 16,1 |
| 44 | $i-C_4H_9-$ | 3 | " | 0 | 15,94 / 16,0 |
| 45 | $C_6H_{11}-$ | 3 | " | 0 | 14,35 / 14,5 |
| 46 | $t-C_4H_9-$ | 3 | 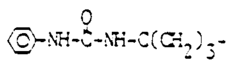 | 0 | 16,08 / 15,8 |
| 47 | $C_6H_5-CH_2-$ | 3 | " | 0 | 14,03 / 14,6 |
| 48 | $n-C_{10}H_{21}-$ | 3 | " | 0 | 11,81 / 12,0 |
| 49 | $C_6H_{11}-$ | 3 | 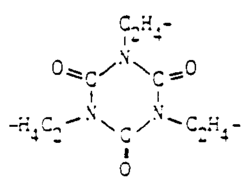 | 0 | 15,84 / 15,7 |
| 50 | $C_6H_5-CH_2-$ | 3 |  | 0 | 15,30 / 15,6 |
| 51 | $t-C_4H_9-$ | 3 | 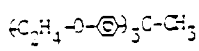 | 0 | 9,39 / 9,1 |

| Nr. | $R^1$ | m | X | n | ber./gef. N% |
|---|---|---|---|---|---|
| 52 | $C_6H_5-O-CH_2-$ | 3 | 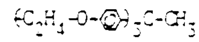 | 0 | 8,04 / 8,5 |
| 53 | $t-C_4H_9-$ | 4 | $C(CH_2)4-$ | 0 | 15,05 / 14,9 |
| 54 | $C_6H_5-CH_2-$ | 4 | " | 0 | 12,72 / 13,0 |
| 55 | $C_6H_{11}-$ | 4 | 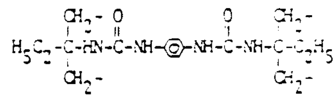 | 0 | 15,11 / 15,0 |
| 56 | $C_6H_5-CH_2-$ | 4 | " | 0 | 14,70 / 15,1 |
| 57 | $t-C_4H_9-$ | 4 | 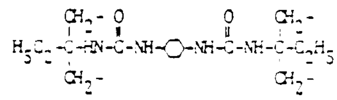 | 0 | 16,43 / 16,8 |
| 58 | $C_6H_{11}-$ | 4 | " | 0 | 15,04 / 15,2 |
| 59 | $C_6H_{11}-$ | 4 | 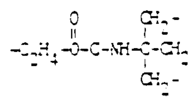 | 0 | 13,98 / 14,2 |
| 60 | $t-C_4H_9-$ | 4 | 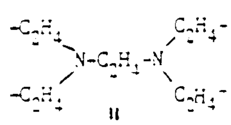 | 0 | 16,58 / 16,5 |
| 61 | $n-C_6H_{13}-$ | 4 | " | 0 | 14,63 / 15,0 |
| 62 | $t-C_4H_9-$ | 5 | 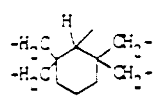 | 0 | 14,28 / 14,5 |
| 63 | $t-C_4H_9-$ | 5 | " | 1 | 13,52 / 12,8 |
| 64 | $t-C_4H_9-$ | 6 | 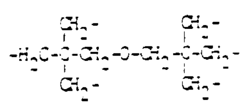 | 0 | 14,40 / 14,7 |
| 65 | $C_6H_{11}-$ | 6 | " | 0 | 12,70 / 12,4 |

| Nr. | $R^1$ | m | X | n | ber./gef. N% |
|---|---|---|---|---|---|
| 66 | $i-C_4H_9-$ | 6 | 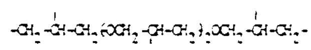 | 0 | 13,70 / 13,2 |
| 67 | $C_4H_7-$ | 6 | " | 0 | 13,52 / 13,2 |
| 68 | $t-C_4H_9-$ | 8 | 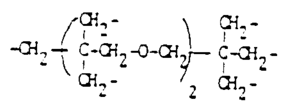 | 0 | 14,10 / 13,8 |
| 69 | $C_6H_{11}-$ | 8 | " | 0 | 12,46 / 12,1 |

$C_4H_7-$ = Cyclobutyl-; $C_6H_{11}-$ = Cyclohexyl-.

Anwendungsbeispiele

Die folgenden Beispiele geben einige Anwendungsmöglichkeiten der erfindungsgemäßen strahlungsempfindlichen Gemische wieder. Diese sollen lediglich erläuternden Charakter haben, jedoch die Erfindung nicht beschränken. In den Beispielen verhalten sich Gewichtsteile (Gt) zu Volumenteilen (Vt) bzw. g:$cm^3$ wie 1:1.

Beispiel 1

Es wird ein strahlungsempfindliches Gemisch hergestellt, enthaltend

0,19 Gt 2,2,2-Tris-[4-(7-cyclohexyl-6-diazo-1,4-dioxa-7,5-dioxo-heptyl)-phenyl]-ethan (Beispiel 6) und
0,75 Gt Poly-(3-methyl-4-hydroxystyrol) in
4,25 Gt Propylenglykolmonomethyletheracetat.

Von dieser Lösung werden 1,5 ml durch einen Filter mit einem Porendurchmesser von 0,2 µm filtriert und auf einen Siliciumwafer mit einem Durchmesser von 7,62 cm aufgetragen. Durch Rotation des Wafers bei 2.000 Upm für 40 sec wird eine homogene Schicht erzeugt, die nach dem Trocknen dieser in einem Umluftofen bei 90° C und für 30 min eine Dicke von 0,87 µm aufweist.

Der in dieser Weise beschichtete Wafer wird durch eine Photomaske hindurch mit der UV-Strahlung einer Xenon-Quecksilberdampflampe bei 260 nm mit einer Energie von 112 mJ/$cm^3$ bildmäßig bestrahlt.

Anschließend wird die bildmäßig bestrahlte Schicht mit einer 0,3 n wäßrigen Lösung von Tetramethylammoniumhydroxid für 120 sec entwickelt. Der Abtrag der Schicht in den unbelichteten Bereichen (Dunkelabtrag) war kleiner als 20 nm. Strukturen, auch mit Dimensionen von kleiner als 1,0 µm, waren bei guter Kantenstabilität saüber aufgelöst.

Beispiel 2

Es wird ein strahlungsempfindliches Gemisch hergestellt, enthaltend

0,19 Gt N-Cylohexyl-N'-tris-[(5-cyclohexyl-4-diazo-3,5-dioxo-2-oxa)-pentyl]-methyl-harnstoff (Beispiel 45) und
0,75 Gt Poly-(3-methyl-4-hydroxystyrol) in
4,25 Gt Propylenglykolmonomethyletheracetat.

Von dieser Lösung wird unter Verwendung der Angabe aus Beispiel 1 ein strahlungsempfindliches Gemisch hergestellt. Analog zu Beispiel 1 wird belichtet und entwickelt. Für die Belichtung wird allerdings eine Belichtungsenergie von 60 mJ/$cm^2$ angewendet. Es konnten kantenscharfe Strukturen erhalten werden.

Beispiel 3

Es wird ein strahlungsempfindliches Gemisch hergestellt, enthaltend

0,19 Gt Tris-[(5-diazo-4,6-dioxo-3-oxa-7-phenyl)-heptyl]-amin (Beispiel 40) und
0,75 Gt Poly-(3-methyl-4-hydroxystyrol) in

4,25 Gt Propylenglykolmonomethyletheracetat,
und entsprechend dem Beispiel 2 weiterverarbeitet. Bei einer bildmäßigen Bestrahlung mit einer Energie von 71 mJ/cm$^2$ konnten kantenscharfe Strukturen erhalten werden.

Beispiel 4

Es wird ein strahlungsempfindliches Gemisch hergestellt, enthaltend
0,19 Gt Tris-[(5-diazo-4,6-dioxo-3-oxa-7-phenyl)-heptyl]-amin und
0,55 Gt Poly-(styrol-co-maleinimid) (1:1) in
4,45 Gt Cyclohexanon.

1,5 ml dieser Lösung werden mittels eines Filters mit einem Porendurchmesser von 0,2 µm filtriert und auf einen mit 2.000 Upm rotierenden Siliciumwafer mit einem Durchmesser von 7,62 cm aufgetragen. Nach 40 sec kann eine homogene Schicht erhalten werden. Nach dem Trocknen der Schicht bei 90° C und für 30 min beträgt die Schichtdicke der homogenen strahlungsempfindlichen Schicht 0,891 µm.

Bei einer Wellenlänge von 260 nm wird der beschichtete Wafer anschließend durch eine Photomaske hindurch bildmäßig belichtet. Als UV-Strahlungsquelle wird eine Xenon-Quecksilberdampflampe verwendet. Die Belichtungsenergie beträgt 200 mJ/cm$^2$.

Mit einer 0,02 n wäßrigen Lösung von Tetramethylammoniumhydroxid konnte die Schicht anschließend innerhalb von 60 sec einwandfrei entwickelt werden. Der Dunkelabtrag war kleiner als 10 nm; 1,0 µm breite Strukturen zeigten bei guter Kantensteilheit eine gute Auflösung.

Beispiele 5 bis 14

Analog zu Beispiel 4, jedoch unter Verwendung der im Folgenden aufgeführten photoaktiven Verbindungen, wurden strahlungsempfindliche Gemische hergestellt und verarbeitet. In allen Fällen wurde eine hohe Auflösung unter Anwendung der angegebenen Belichtungsenergie erreicht.

| Beispiel | photoaktive Komponente | Belichtungsenergie [mJ/cm$^2$] |
|---|---|---|
| 5 | Bis-[N,N'(5-diazo-6-cyclohexyl-4,6-dioxo-3-oxa)-hexyl]piperazin (Beispiel 32) | 60 |
| 6 | Bis-4,13-diazo-6,11-dioxa-2,2,15,15-tetramethyl-3,5,12,14-tetraoxo-hexadec-8-in (Beispiel 2) | 45 |
| 7 | Tris-[(5-diazo-7,7-dimethyl-4,6-dioxo-3-oxa)-octyl]-amin (Beispiel 4) | 70 |
| 8 | Tris-[(6-cyclohexyl-5-diazo-4,6-dioxo-3-oxa)-hexyl]-amin (Beispiel 38) | 110 |
| 9 | Tris-[(5-diazo-3,11-dioxa-4,6,10-trioxo)-tridecyl]-amin (Beispiel 42) | 130 |

| Beispiel | photoaktive Komponente | Belichtungsenergie mJ/cm² |
|---|---|---|
| 10 | Tris-[(5-diazo-4,6-dioxo-3-oxa-7-phenyl)-heptyl]-amin (Beispiel 40) | 200 |
| 11 | Tris-[(5-diazo-3,8-dioxa-4,6-dioxo-8-phenyl)-octyl]-amin | 230 |
| 12 | N-[1,1-Bis-(5-cyclohexyl-4-diazo-3,5-dioxo-2-oxa- pentyl)-6-cyclohexyl-5-diazo-4,6-dioxo-3-oxa-hexyl]-O-[6-cyclohexyl-5-diazo-4,6-dioxo-3-oxa-hexyl)-urethan (Beispiel 7) | 60 |
| 13 | 1,19-Bis-cyclohexyl-2,18-bis-diazo-6,14-[bis-(4-cyclohexyl-3-diazo-2,4-dioxo-1-oxa)-butyl]-10-[8-(cyclohexyl-7-diazo-1,5-dioxa-6,8-dioxo-3-(3-cyclohexyl-3-diazo-2,4-dioxo-1-oxa-butyl)) -1,5-dioxa-6,8-dioxo-octyl]-4,8,12,16-tetraoxa-1,3,17,19-tetraoxo-nonadecan (Beispiel 9) | 96 |
| 14 | Gemisch aus gleichen Gewichtsteilen an Tris-[(5-diazo-7,7-dimethyl-4,6-dioxo-3-oxa)-heptyl]-amin (Beispiel 4) und Tris-[(4-(7-cyclohexyl-6-diazo-1,4-dioxa-5,7-dioxo)-heptyl)-phenyl]-ethan (Beispiel 6) | 164 |

## Beispiel 15

Es wird ein strahlungsempfindliches Gemisch hergestellt, enthaltend

| | |
|---|---|
| 0,19 Gt | der Verbindung (25) aus dem Herstellungsbeispiel 10 und |
| 0,55 Gt | Poly-(styrol-co-maleinimid) (1:1) in |
| 4,45 Gt | Cyclohexanon. |

Von der Lösung werden 1,5 ml durch einen Filter mit einem Porendurchmesser von 0,2 µm auf einen Siliciumwafer mit einem Durchmesser von 7,62 cm aufgeschleudert und durch Rotation des Wafers mit 4.000 Upm innerhalb von 40 Sekunden eine homogene Schicht erzeugt. Die Schichtdicke nach dem Trocknen bei 90° C für 30 Minuten in einem Umluftofen beträgt 0,87 µm.

Der beschichtete Wafer wird durch eine Photomaske hindurch mit UV-Strahlung einer Xenon-Quecksilberdampflampe im Bereich von 260 nm mit einer Belichtungsenergie von 200 mJ/cm² bestrahlt. Anschließend wird mit einer 0,3 n wäßrigen Lösung von Tetraethylammoniumhydroxid innerhalb von 60 Sekunden entwickelt. Der Dunkelabtrag war kleiner als 2 nm/min. Bei Anwendung einer Belichtungsenergie von 116 mJ/cm² konnten

1 µm-Strukturen sauber und kantenscharf aufgelöst werden.

Beispiel 16 (Vergleichsbeispiel)

Es wird ein strahlungsempfindliches Gemisch hergestellt, enthaltend

0,19 Gt der Verbindung (26) aus dem Vergleichsbeispiel 11 und
0,55 Gt Poly-(styrol-co-maleinimid) (1:1) in
4,45 Gt Cyclohexanon.

Das Gemisch wird analog zu Beispiel 15 verarbeitet. Es konnte allerdings festgestellt werden, daß nach einer Entwicklungszeit von 60 sec mit dem gleichen Entwickler aus Beispiel 15 sowohl die belichteten wie auch unbelichteten Bereiche vollständig entfernt worden waren. Der Abtrag der unbelichteten Bereiche (Dunkelabtrag) war mit 1.660 nm/min vergleichbar mit der Abtragsrate für die belichteten Bereiche (2.360 nm/min). Lediglich bei Anwendung der Entwicklungszeit von 20 Sekunden konnten Strukturen, allerdings mit sehr flachen Kanten, erzeugt werden.

**Patentansprüche**

1. Positiv arbeitendes strahlungsempfindliches Gemisch, enthaltend im wesentlichen eine photoaktive Komponente und ein in Wasser unlösliches, in wäßrig alkalischer Lösung lösliches, zumindest aber quellbares Bindemittel, dadurch gekennzeichnet, daß als photoaktive Komponente ein mehrfunktioneller α-Diazo-β-Ketoester der allgemeinen Formel I verwendet wird

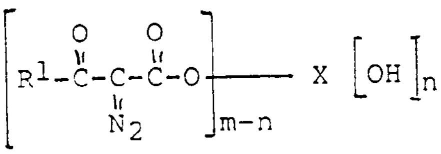

(I),

worin

$R^1$ einen aliphatischen, cycloaliphatischen oder araliphatischen oder aromatischen Rest mit 4 bis 20 Kohlenstoffatomen, wobei einzelne $CH_2$-Gruppen durch Sauerstoff- oder Schwefelatome oder durch

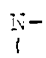

oder NH-Gruppen ersetzt sein können und/oder Ketogruppen enthalten,

X einen aliphatischen, cycloaliphatischen, carbocyclischen, heterocyclischen oder araliphatischen Rest mit 2 bis 22 Kohlenstoffatome, wobei einzelne $CH_2$-Gruppen durch Sauerstoff- oder Schwefelatome oder durch die Gruppen $-NR^2-$, -C(O)-O-, $-C(O)-NR^2-$,

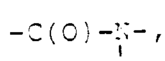

$-NR^2-C(O)-NR^3-$, $-O-C(O)-NR^2-$,

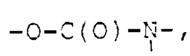

-O-C(O)-O- oder CH-Gruppen durch

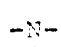

ersetzt sein können, worin $R^2$ und $R^3$ voneinander unabhängig für Wasserstoff oder einen aliphatischen, carbocyclischen oder araliphatischen Rest stehen,

m eine ganze Zahl von 2 bis 10 und

n eine ganze Zahl von 0 bis 2

bedeuten, mit der Maßgabe, daß

$m-n \geqq 2$ ist.

2. Positiv arbeitendes strahlungsempfindliches Gemisch nach Anspruch 1, dadurch gekennzeichnet, daß $R_2$ und $R_3$ Wasserstoff, $(C_1$-$C_3)$Alkyl, $(C_6$-$C_{12})$Aryl oder $(C_6$-$C_{11})$Aralkyl, insbesondere $(C_1$-$C_3)$Alkyl oder Wasserstoff bedeuten, wobei diese, insbesondere Aryl oder Aralkyl, am Kern durch Alkyl, Alkoxy, Halogen oder Amino substituiert sein können.

3. Positiv arbeitendes strahlungsempfindliches Gemisch nach den Ansprüchen 1 oder 2, dadurch gekennzeichnet, daß $R^1$ und/oder X substituiert sind durch $(C_1$-$C_3)$Alkyl, $(C_1$-$C_3)$Alkoxy, Halogen, Amino oder Nitrilo, insbesondere durch $(C_1$-$C_3)$Alkyl oder $(C_1$-$C_3)$Alkoxy.

4. Positiv arbeitendes strahlungsempfindliches Gemisch nach einem oder mehreren der Ansprüche 1 bis 3, dadurch gekennzeichnet, daß sofern $R^1$ einen aliphatischen Rest darstellt und dieser substituiert ist, dieser insbesondere 4 bis 8, Kettenglieder aufweist.

5. Positiv arbeitendes strahlungsempfindliches Gemisch nach Anspruch 4, dadurch gekennzeichnet, daß $CH_2$-Gruppen insbesondere durch Sauerstoffatome, -NH-Gruppen oder Ketogruppen ersetzt sind und diese Reste daher Ether-, Keto-, Ester-, Amido- und/oder Imidogruppen enthalten.

6. Positiv arbeitendes strahlungsempfindliches Gemisch nach einem oder mehreren der Ansprüche 1 bis 3, dadurch gekennzeichnet, daß sofern $R^1$ einen aliphatischen Rest darstellt und dieser unsubstituiert ist, dieser bis zu 20 Kettenglieder aufweisen kann, wobei insbesondere der t-Butylrest bevorzugt ist.

7. Positiv arbeitendes strahlungsempfindliches Gemisch nach einem oder mehreren der Ansprüche 1 bis 3, dadurch gekennzeichnet, daß $R^1$ einen cycloaliphatischen Rest bedeutet mit 4, 5, 6 oder 12, insbesondere 4, 5 oder 6, besonders bevorzugt 6, Ringgliedern, und der insbesondere unsubstituiert ist.

8. Positiv arbeitendes strahlungsempfindliches Gemisch nach einem oder mehreren der Ansprüche 1 bis 3, dadurch gekennzeichnet, daß $R^1$ ein araliphatischer Rest ist mit 2 bis 11, insbesondere 2 bis 5, Kettengliedern im aliphatischen Teil des Restes.

9. Positiv arbeitendes strahlungsempfindliches Gemisch nach Anspruch 8, dadurch gekennzeichnet, daß der aliphatische Teil des Restes $R^1$ 1 oder 2 Kettenglieder enthält, sofern dieser Teil eine reine Kohlenstoffkette aufweist.

10. Positiv arbeitendes strahlungsempfindliches Gemisch nach Anspruch 8, dadurch gekennzeichnet, daß der aliphatische Teil des Restes $R^1$ 3 bis 5 Kettenglieder aufweist, sofern in diesem Teil $CH_2$-Gruppen durch Heteroatome ersetzt sind.

11. Positiv arbeitendes strahlungsempfindliches Gemisch nach einem oder mehreren der Ansprüche 1 bis 10, dadurch gekennzeichnet, daß im Rest X maximal 5, insbesondere maximal 3 $CH_2$-Gruppen durch die Heteroatome bzw. die genannten Gruppen ersetzt sind.

12. Positiv arbeitendes strahlungsempfindliches Gemisch nach einem oder mehreren der Ansprüche 1 bis 10, dadurch gekennzeichnet, daß der Rest X aliphatisch und unsubstituiert ist und maximal 6 Kohlenstoffatome aufweist.

13. Positiv arbeitendes strahlungsempfindliches Gemisch nach Anspruch 12, dadurch gekennzeichnet, daß dieser maximal eine C-C-Mehrfachbindung aufweist.

14. Positiv arbeitendes strahlungsempfindliches Gemisch nach Anspruch 11, dadurch gekennzeichent, daß die $CH_2$-Gruppen ersetzenden Heteroatome von einem Typ sind.

15. Positiv arbeitendes strahlungsempfindliches Gemisch nach Anspruch 11, dadurch gekennzeichnet, daß sofern eine CH-Gruppe durch

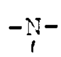

ersetzt ist, keine weitere Substitution im Rest X vorliegt.

16. Positiv arbeitendes strahlungsempfindliches Gemisch nach Anspruch 11, dadurch gekennzeichnet, daß X ein cycloaliphatischer Rest ist, in dem der cycloaliphatische Teil unsubstituiert ist und benachbart ist

einer $CH_2$-Gruppe des aliphatischen Teils, die substituiert ist durch ein Heteroatom oder eine Gruppe des Anspruchs 1.

**17.** Positiv arbeitendes strahlungsempfindliches Gemisch nach Anspruch 16, dadurch gekennzeichnet, daß der cycloaliphatische Teil direkt benachbart ist zu einem Stickstoffatom, insbesondere der Gruppen

$$-NH-\overset{O}{\overset{\|}{C}}-O-$$

oder

$$-NH-\overset{O}{\overset{\|}{C}}-NH-.$$

**18.** Positiv arbeitendes strahlungsempfindliches Gemisch nach Anspruch 16, dadurch gekennzeichnet, daß der cycloaliphatische Teil über eine Ethylengruppe mit dem Sauerstoffatom eines

$$-NH-\overset{O}{\overset{\|}{C}}-O-$$

Restes verknüpft ist.

**19.** Positiv arbeitendes strahlungsempfindliches Gemisch nach Anspruch 11, dadurch gekennzeichnet, daß X ein araliphatischer Rest ist, wobei der aromatische Teil insbesondere einen Phenyl- bzw. Phenylenrest darstellt, der über ein Heteroatom mit dem aliphatischen Teil dieses Restes verknüpft ist.

**20.** Positiv arbeitendes strahlungsempfindliches Gemisch nach einem oder mehreren der Ansprüche 1 bis 19, dadurch gekennzeichnet, daß m eine ganze Zahl von 2 bis 8 und n eine ganze Zahl von 0 bis 2 ist.

**21.** Positiv arbeitendes strahlungsempfindliches Gemisch nach Anspruch 20, dadurch gekennzeichnet daß m eine ganze Zahl von 2 bis 6 und n = 0 ist.

**22.** Positiv arbeitendes strahlungsempfindliches Gemisch nach einem oder mehreren der Ansprüche 1 bis 21, dadurch gekennzeichnet, daß 1 mehrere, insbesondere 2, $\alpha$-Diazo-$\beta$-ketoester der allgemeinen Formel I im strahlungsempfindlichen Gemisch enthalten sind.

**23.** Positiv arbeitendes strahlungsempfindliches Gemisch nach einem oder mehreren der Ansprüche 1 bis 22, dadurch gekennzeichnet, daß die Konzentration der photoaktiven Komponenten im strahlungsempfindlichen Gemisch 4 bis 40 Gew.-%, insbesondere 6 bis 30 Gew.-%, beträgt.

**24.** Positiv arbeitendes strahlungsempfindliches Gemisch nach einem oder mehreren der Ansprüche 1 bis 23, dadurch gekennzeichnet, daß das Bindemittel im Wellenlängenbereich von 190 bis 300 nm eine geringe Eigenabsorption aufweist.

**25.** Positiv arbeitendes strahlungsempfindliches Gemisch nach einem oder mehreren der Ansprüche 1 bis 24, dadurch gekennzeichnet, daß das Bindemittel bis zu 30 Gew.-%, insbesondere bis zu 20 Gew.-%, ein Novolak-Kondensationsharz enthält.

**26.** Positiv arbeitendes strahlungsempfindliches Gemisch nach einem oder mehreren der Ansprüche 1 bis 25, dadurch gekennzeichnet, daß das Bindemittel phenolische Hydroxylgruppen enthält.

**27.** Positiv arbeitendes strahlungsempfindliches Gemisch nach einem oder mehreren der Ansprüche 1 bis 26, dadurch gekennzeichnet, daß das Bindemittel in einer Konzentration von 60 bis 96 Gew.-%, insbesondere 70 bis 94 Gew.-%, im strahlungsemgfindlichen Gemisch enthalten ist.

**28.** Positiv arbeitendes Aufzeichnungsmaterial enthaltend einen Träger und ein darauf aufgetragenes positiv arbeitendes Gemisch, dadurch gekennzeichnet, daß das positiv arbeitende Gemisch ein Gemisch entsprechend einem oder mehreren der Ansprüche 1 bis 27 entspricht.

**29.** Verfahren zur Herstellung eines positiv arbeitenden Aufzeichnungsmaterials nach Anspruch 28, dadurch gekennzeichnet, daß der ggf. mit einem Haftvermittler vorbehandelte Träger durch Aufsprühen, Walzen, Rakeln, Gießen, durch Fließbeschichten, Schleuderbeschichten und Tauchbeschichten oder mittels Breitschlitzdüsen mit dem strahlungsempfindlichen Gemisch beschichtet wird und das Lösungsmittel durch Verdampfen entfernt wird, so daß eine Schichtstärke von 0,1 bis 100 μm, insbesondere von 0,3 bis 10 μm, entsteht.

**30.** Verfahren nach Anspruch 29, dadurch gekennzeichnet, daß das Aufzeichnungsmaterial mit Licht einer Wellenlänge von 190 bis 300 nm bildmäßig belichtet wird und das entstehende Bild mittels einer wäßrigen Entwicklungslösung freigelegt wird.

**31.** Verfahren nach den Ansprüchen 29 oder 30, dadurch gekennzeichnet, daß die entstandene Struktur durch Erhitzen und/oder durch ganzflächige Belichtung nachgehärtet wird.

**32.** Verfahren nach Anspruch 29, dadurch gekennzeichnet, daß das strahlungsempfindliche Gemisch auf einen Zwischenträger aufgetragen wird, und von dem erst auf den endgültigen Träger auflaminiert wird.

## Claims

**1.** A positively operating radiation-sensitive mixture containing essentially a photoactive component and a binder which is insoluble in water but soluble or at least swellable in aqueous alkaline solution, in which a polyfunctional α-diazo-β-keto ester of the general formula I

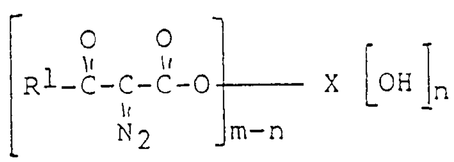

(I)

in which

$R^1$ denotes an aliphatic, cycloaliphatic or araliphatic or aromatic radical having 4 to 20 carbon atoms, in which individual $CH_2$ groups can be replaced by oxygen or sulfur atoms or by

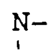

or NH groups and/or contain keto groups,

X denotes an aliphatic, cycloaliphatic, carbocyclic, heterocyclic or araliphatic radical having 2 to 22 carbon atoms, in which individual $CH_2$ groups can be replaced by oxygen or sulfur atoms or by the groups $-NR^2-$, -C(O)-O-, $-C(O)-NR^2-$,

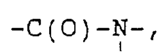

$-NR^2-C(O)-NR^3-$, $-O-C(O)-NR^2-$,

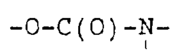

or -O-C(O)-O-, or CH groups can be replaced by

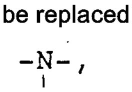

in which $R^2$ and $R^3$ independently of one another represent hydrogen or an aliphatic, carbocyclic or araliphatic radical,

m denotes an integer from 2 to 10 and

n denotes an integer from 0 to 2,

with the proviso that
m-n is ≧ 2.
is used as the photoactive component.

2. A positively operating radiation-sensitive mixture as claimed in claim 1, in which $R_2$ and $R_3$ denote hydrogen, $(C_1-C_3)$alkyl, $(C_5-C_{12})$aryl or $(C_6-C_{11})$aralkyl, in particular $(C_1-C_3)$alkyl or hydrogen, it being possible for these, in particular aryl or aralkyl, to be substituted on the nucleus by alkyl, alkoxy, halogen or amino.

3. A positively operating radiation-sensitive mixture as claimed in either of claims 1 and 2, in which $R^1$ and/or X are substituted by $(C_1-C_3)$alkyl, $(C_1-C_3)$alkoxy, halogen, amino or nitrilo, in particular by $(C_1-C_3)$alkyl or $(C_1-C_3)$alkoxy.

4. A positively operating radiation-sensitive mixture as claimed in any one of claims 1 to 3, in which, if $R^1$ represents an aliphatic radical and this is substituted, this has, in particular, 4 to 8 chain members.

5. A positively operating radiation-sensitive mixture as claimed in claim 4, in which $CH_2$ groups are replaced in particular by oxygen atoms, -NH- groups or keto groups and these radicals therefore contain ether, keto, ester, amido and/or imido groups.

6. A positively operating radiation-sensitive mixture as claimed in any one of claims 1 to 3, in which, if $R^1$ represents an aliphatic radical which is unsubstituted, it can contain up to 20 chain members, the t-butyl radical being particularly preferred.

7. A positively operating radiation-sensitive mixture as claimed in any one of claims 1 to 3, in which $R^1$ denotes a cycloaliphatic radical having 4, 5, 6 or 12, in particular 4, 5 or 6, particularly preferably 6, ring members which is, in particular, unsubstituted.

8. A positively operating radiation-sensitive mixture as claimed in any one of claims 1 to 3, in which $R^1$ is an araliphatic radical having 2 to 11, in particular 2 to 5, chain members in the aliphatic part of the radical.

9. A positively operating radiation-sensitive mixture as claimed in claim 8, in which the aliphatic part of the radical $R^1$ contains 1 or 2 chain members, if this part contains a pure carbon chain.

10. A positively operating radiation-sensitive mixture as claimed in claim 8, in which the aliphatic part of the radical $R^1$ contains 3 to 5 chain members if $CH_2$ groups in this part are replaced by hetero atoms.

11. A positively operating radiation-sensitive mixture as claimed in any one of claims 1 to 10, in which not more than 5, in particular not more than 3, $CH_2$ groups in the radical X are replaced by the hetero atoms or the groups mentioned.

12. A positively operating radiation-sensitive mixture as claimed in any one of claims 1 to 10, in which the radical X is aliphatic and unsubstituted and contains not more than 6 carbon atoms.

13. A positively operating radiation-sensitive mixture as claimed in claim 12, in which this radical contains not more than one C-C multiple bond.

14. A positively operating radiation-sensitive mixture as claimed in claim 11, in which the hetero atoms replacing $CH_2$ groups are of one type.

15. A positively operating radiation-sensitive mixture as claimed in claim 11, in which, if a CH group is replaced by

$$-\underset{|}{N}-,$$

no further substitution is present in the radical X.

16. A positively operating radiation-sensitive mixture as claimed in claim 11, in which X is a cycloaliphatic radical in which the cycloaliphatic part is unsubstituted and is adjacent to a $CH_2$ group of the aliphatic part, which is substituted by a hetero atom or by a group as claimed in claim 1.

**17.** A positively operating radiation-sensitive mixture as claimed in claim 16, in which the cycloaliphatic part is directly adjacent to a nitrogen atom, in particular of the group

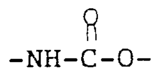 or 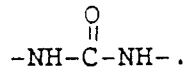.

**18.** A positively operating radiation-sensitive mixture as claimed in claim 16, in which the cycloaliphatic part is linked via an ethylene group to the oxygen atom of a

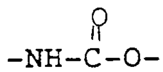

radical.

**19.** A positively operating radiation-sensitive mixture as claimed in claim 11, in which X is an araliphatic radical, the aromatic part representing, in particular, a phenyl or phenylene radical which is linked via a hetero atom to the aliphatic part of this radical.

**20.** A positively operating radiation-sensitive mixture as claimed in any one of claims 1 to 19, in which m is an integer from 2 to 8 and n is an integer from 0 to 2.

**21.** A positively operating radiation-sensitive mixture as claimed in claim 20, in which m is an integer from 2 to 6 and n is 0.

**22.** A positively operating radiation-sensitive mixture as claimed in any one of claims 1 to 21, in which the radiation-sensitive mixture contains several, in particular 2, α-diazo-β-keto esters of the general formula I.

**23.** A positively operating radiation-sensitive mixture as claimed in any one of claims 1 to 22, in which the concentration of the photoactive components in the radiation-sensitive mixture is 4 to 40% by weight, in particular 6 to 30% by weight.

**24.** A positively operating radiation-sensitive mixture as claimed in any one of claims 1 to 23, in which the binder has a low intrinsic absorption in the wavelength range from 190 to 300 nm.

**25.** A positively operating radiation-sensitive mixture as claimed in any one of claims 1 to 24, in which the binder contains up to 30% by weight, in particular up to 20% by weight, of a novolak condensation resin.

**26.** A positively operating radiation-sensitive mixture as claimed in any one of claims 1 to 25, in which the binder contains phenolic hydroxyl groups.

**27.** A positively operating radiation-sensitive mixture as claimed in any one of claims 1 to 26, in which the binder is contained in the radiation-sensitive mixture in a concentration of 60 to 96% by weight, in particular 70 to 94% by weight.

**28.** A positively operating recording material containing a carrier and a positively operating mixture applied thereto, in which the positively operating mixture corresponds to a mixture as claimed in any one of claims 1 to 27.

**29.** A process for the preparation of a positively operating recording material as claimed in claim 28, which comprises coating the carrier, if appropriate pretreated with an adhesion promoter, with the radiation-sensitive mixture by spraying, rolling, knife-coating, pouring, flow-coating, whirler-coating or dip-coating or by means of a slot die, and removing the solvent by evaporation, so that a layer thickness of 0.1 to 100 μm, in particular 0.3 to 10 μm, is formed.

**30.** The process as claimed in claim 29, wherein the recording material is exposed imagewise to light of wavelength 190 to 300 nm and the image formed is set free by means of an aqueous development solution.

31. The process as claimed in either of claims 29 or 30, wherein the structure formed is postcured by heating and/or by exposure over the entire area.

32. The process as claimed in claim 29, wherein the radiation-sensitive mixture is applied to an intermediate carrier and laminated from the first onto the final carrier.

## Revendications

1. Composition sensible aux radiations, travaillant en positif, contenant essentiellement un composant photoactif et un liant insoluble dans l'eau, soluble ou au moins susceptible de gonfler en solution aqueuse-alcaline, caractérisée en ce que, en tant que composant photoactif, on utilise un α-diazo-β-cétoester polyfonctionnel de formule générale I

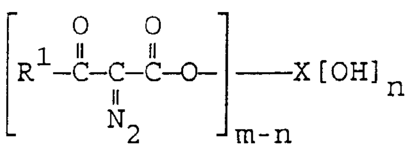 (I)

dans laquelle

R¹ représente un radical aliphatique, cycloaliphatique ou araliphatique ou aromatique ayant de 4 à 20 atomes de carbone, des groupes $CH_2$ individuels pouvant être remplacés par des atomes d'oxygène ou de soufre ou par

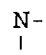

ou des groupes NH et/ou comprenant des groupes céto,

X représente un radical aliphatique, cycloaliphatique, carbocyclique, hétérocyclique ou araliphatique ayant de 2 à 22 atomes de carbone, des groupes $CH_2$ individuels pouvant être remplacés par des atomes d'oxygène ou de soufre ou par les groupes $-NR^2-$, -C(O)-O-, $-C(O)-NR^2-$,

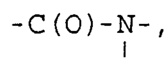

$-NR^2-C(O)-NR^3-$, $-O-C(O)-NR^2-$,

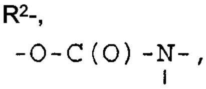

-O-C(O)-O-, ou des groupes CH pouvant être remplacés par

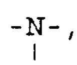

$R^2$ et $R^3$ représentant, indépendamment l'un de l'autre, un atome d'hydrogène ou un radical aliphatique, carbocyclique ou araliphatique,

m représente un nombre entier allant de 2 à 10, et

n représente un nombre entier allant de 0 à 2,

étant entendu que $m - n \geqq 2$.

2. Composition sensible aux radiations, travaillant en positif, selon la revendication 1, caractérisée en ce que $R^2$ et $R^3$ représentent un atome d'hydrogène ou un groupe alkyle en $C_1$-$C_3$, aryle en $C_6$-$C_{12}$ ou aralkyle en $C_6$-$C_{11}$, en particulier un groupe alkyle en $C_1$-$C_3$ ou un atome d'hydrogène, ces groupes, en particulier les groupes aryle ou aralkyle, pouvant être substitués sur le noyau par des atomes d'halogène ou par des groupes alkyle, alcoxy ou amino.

3. Composition sensible aux radiations, travaillant en positif, selon la revendication 1 ou 2, caractérisée en ce que $R^1$ et/ou X est(sont) substitué(s) par des atomes d'halogène ou par des groupes alkyle en $C_1$-$C_3$, alcoxy en $C_1$-$C_3$, amino ou nitrilo, en particulier par des groupes alkyle en $C_1$-$C_3$ ou alcoxy en $C_1$-$C_3$.

4. Composition sensible aux radiations, travaillant en positif, selon une ou plusieurs des revendication 1 à 3, caractérisée en ce que, lorsque $R^1$ représente un radical aliphatique et celui-ci est substitué, il comporte en particulier 4 à 8 chaînons.

5. Composition sensible aux radiations, travaillant en positif, selon la revendication 4, caractérisée en ce que des groupes $CH_2$ sont remplacés en particulier par des atomes d'oxygène, des groupes -NH- ou des groupes céto, et ces radicaux par conséquent contiennent des groupes éther, céto, ester, amido et/ou imido.

6. Composition sensible aux radiations, travaillant en positif, selon une ou plusieurs des revendication 1 à 3, caractérisée en ce que, lorsque $R^1$ représente un radical aliphatique et celui-ci est non substitué, il peut comporter jusqu'à 20 chaînons, en particulier le radical tert-butyle étant préféré.

7. Composition sensible aux radiations, travaillant en positif, selon une ou plusieurs des revendications 1 à 3, caractérisée en ce que $R^1$ représente un radical cycloaliphatique à 4, 5, 6 ou 12, en particulier à 4, 5 ou 6, de façon particulièrement préférée 6 chaînons de cycle, et qui en particulier n'est pas substitué.

8. Composition sensible aux radiations, travaillant en positif, selon une ou plusieurs des revendications 1 à 3, caractérisée en ce que $R^1$ est un radical araliphatique ayant de 2 à 11, en particulier de 2 à 5 chaînons dans le fragment aliphatique du radical.

9. Composition sensible aux radiations, travaillant en positif, selon la revendication 8, caractérisée en ce que le fragment aliphatique du radical $R^1$ contient 1 ou 2 chaînons, dans la mesure où ce fragment comporte une chaîne purement carbonée.

10. Composition sensible aux radiations, travaillant en positif, selon la revendication 8, caractérisée en ce que le fragment aliphatique du radical $R^1$ comporte 3 à 5 chaînons, dans la mesure où dans ce fragment des groupes $CH_2$ sont remplacés par des hétéroatomes.

11. Composition sensible aux radiations, travaillant en positif, selon une ou plusieurs des revendications 1 à 10, caractérisée en ce que, dans le radical X, au maximum 5, en particulier au maximum 3 groupes $CH_2$ sont remplacés par les hétéroatomes ou les groupes cités.

12. Composition sensible aux radiations, travaillant en positif, selon une ou plusieurs des revendications 1 à 10, caractérisée en ce que le radical X est aliphatique et non substitué et comporte au maximum 6 atomes de carbone.

13. Composition sensible aux radiations, travaillant en positif, selon la revendication 12, caractérisée en ce qu'elle comporte au maximum une liaison multiple C-C.

14. Composition sensible aux radiations, travaillant en positif, selon la revendication 11, caractérisée en ce que les hétéroatomes remplaçant des groupes $CH_2$ sont d'un seul type.

15. Composition sensible aux radiations, travaillant en positif, selon la revendication 11, caractérisée en ce que, lorsqu'un groupe $CH_2$ est remplacé par

-N-,
 |

il n'y a aucune autre substitution sur le radical X.

16. Composition sensible aux radiations, travaillant en positif, selon la revendication 11, caractérisée en ce que X est un radical cycloaliphatique dans lequel le fragment cycloaliphatique n'est pas substitué et est voisin d'un groupe $CH_2$ du fragment aliphatique qui est substitué par un hétéroatome ou par un groupe de la revendication 1.

17. Composition sensible aux radiations, travaillant en positif, selon la revendication 16, caractérisée en ce que le fragment cycloaliphatique est immédiatement voisin d'un atome d'azote, en particulier des groupes

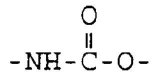

ou

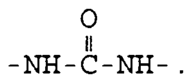

**18.** Composition sensible aux radiations, travaillant en positif, selon la revendication 16, caractérisée en ce que le fragment cycloaliphatique est lié, par l'intermédiaire d'un groupe éthylène, à l'atome d'oxygène d'un radi cal

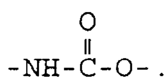

**19.** Composition sensible aux radiations, travaillant en positif, selon la revendication 11, caractérisée en ce que X est un radical araliphatique, le fragment aromatique représentant en particulier un radical phényle ou phénylène, qui est lié, par l'intermédiaire d'un hétéroatome, au fragment aliphatique de ce radical.

**20.** Composition sensible aux radiations, travaillant en positif, selon une ou plusieurs des revendications 1 à 19, caractérisée en ce que m est un nombre entier allant de 2 à 8, et n est un nombre entier allant de 0 à 2.

**21.** Composition sensible aux radiations, travaillant en positif, selon la revendication 20, caractérisée en ce que m est un nombre entier allant de 2 à 6, et n = 0.

**22.** Composition sensible aux radiations, travaillant en positif, selon une ou plusieurs des revendications 1 à 21, caractérisée en ce que plusieurs, en particulier de 2, α-diazo-β-cétoesters de formule générale I, sont contenu dans la composition sensible aux radiations.

**23.** Composition sensible aux radiations, travaillant en positif, selon une ou plusieurs des revendications 1 à 22, caractérisée en ce que la concentration des composants photoactifs dans la composition sensible aux radiations va de 4 à 40 % en poids, en particulier de 6 à 30 % en poids.

**24.** Composition sensible aux radiations, travaillant en positif, selon une ou plusieurs des revendications 1 à 23, caractérisée en ce que le liant présente une faible absorption propre dans la région de longueurs d'onde allant de 190 à 300 nm.

**25.** Composition sensible aux radiations, travaillant en positif, selon une ou plusieurs des revendications 1 à 24, caractérisée en ce que le liant contient jusqu'à 30 % en poids, en particulier jusqu'à 20 % en poids, d'une résine de condensation de type Novolaque.

**26.** Composition sensible aux radiations, travaillant en positif, selon une ou plusieurs des revendications 1 à 25, caractérisée en ce que le liant contient des groupes hydroxy phénoliques.

**27.** Composition sensible aux radiations, travaillant en positif, selon une ou plusieurs des revendications 1 à 26, caractérisée en ce que dans la composition sensible aux radiations le liant est contenu à une concentration de 60 à 96 % en poids, en particulier de 70 à 94 % en poids.

**28.** Matériau de reprographie travaillant en positif, contenant un support et une composition travaillant en positif appliquée sur celui-ci, caractérisé en ce que la composition travaillant en positif correspond à une composition selon une ou plusieurs des revendications 1 à 27.

**29.** Procédé pour la production d'un matériau de reprographie travaillant en positif, selon la revendication 28, caractérisé en ce que le support, éventuellement prétraité par un agent favorisant l'adhérence, est revêtu avec la composition photosensible, par pulvérisation, application au rouleau, enduction à la racle, coulage,

par enduction par ruissellement, enduction par projection centrifuge ou enduction par immersion ou au moyen de filières plates, et le solvant est éliminé par évaporation, de sorte qu'il en résulte une épaisseur de couche de 0,1 à 100 µm, en particulier de 0,3 à 10 µm.

**30.** Procédé selon la revendication 29, caractérisé en ce que le matériau de reprographie est insolé selon l'image avec de la lumière d'une longueur d'onde de 190 à 300 nm, et l'image résultante est dégagée à l'aide d'une solution aqueuse de développement.

**31.** Procédé selon la revendication 29 ou 30, caractérisé en ce que la structure obtenue est post-durcie par chauffage et/ou par insolation de toute la surface.

**32.** Procédé selon la revendication 29, caractérisé en ce que la composition sensible aux radiations est appliquée sur un support intermédiaire et, de celui-ci, seulement alors appliquée par laminage sur le support final.